# EUROPEAN PATENT APPLICATION

(11) **EP 3 875 078 A1**
(43) Date of publication of application: **08.09.2021**
(21) Application number: 20161603.4
(22) Date of filing: 06.03.2020
(51) Int. Cl.: A61K 31/047, A61K 31/415, A61K 31/4184, A61K 31/4709, A61K 31/472, A61K 31/48, A61K 31/4985, A61K 31/502, A61K 31/506, A61K 31/58, A61P 31/14

(54) **COMPOUNDS FOR THE TREATMENT OF COVID-19**

(71) Applicant: Dompe' Farmaceutici S.P.A., 20122 Milan (IT)
(72) Inventor: BECCARI, Andrea Rosario, 80131 Napoli (IT); MANELFI, Candida, 80131 Napoli (IT); TALARICO, Carmine, 80131 Napoli (IT)
(74) Representative: Mauri, Elisabetta Maria Ester

(57) **Abstract**

The present invention relates to compounds that are able to inhibt 3CL protease of COVID-19 virus, SARS-Cov-2

## Description

### FIELD OF THE INVENTION

The present invention relates to compounds that are able to inhibit the 3C-like protease (3CLP) of COVID-19 virus, SARS-CoV-2.

### STATE OF THE ART

Coronaviruses (Covs) are a large family of viruses belonging to the family Coronaviridae. The limited number of coronaviruses known to be circulating in humans were considered to cause mild infections and they were regarded in the past as relatively harmless respiratory human pathogens. However, in the last years the emergence of the severe acute respiratory syndrome coronavirus (SARS-CoV) and the Middle East Respiratory Syndrome (MERS) virus revealed that some coronaviruses can cause severe and sometimes fatal respiratory tract infections in humans (Pereira, H et al., 1989 Coronaviridae. Andrewes 'Viruses of Vertebrates, 5th ed.pp. 42-57; Holmes, K.V. et al.,1996. Virology 1,1075-1093). The first known case of SARS-CoV occurred in Foshan, China in November 2002 and new cases emerged in mainland China in February 2003. The first emergence of MERS-CoV occurred in June 2012 in Saudi Arabia. These events demonstrated that the threats of CoVs should not be underestimated and that it is of paramount importance to advance the knowledge on the replication of these viruses and their interactions with the hosts to develop treatments and vaccines.

In December 2019, atypical pneumonia cases emerged in China and the cause was identified as being a novel coronavirus named SARS-CoV-2 by WHO.

Investigations of the epidemiological and clinical characteristics, and outcomes of patients infected by SARS-CoV-2 demonstrated that the infection caused clusters of severe respiratory illness similar to the known SARS-CoV. Furthermore, it was demonstrated that the SARS-CoV-2 can cause severe illness in some patients, even initially it did not transmit readily between people. However, more recent epidemiological data suggest the new virus has undergone human host adaptation/evolution and has become more efficient in human-to-human transmission. Analysis of SARS-CoV-2 genome sequences obtained from patients during the beginning of the outbreak demonstrated that they are almost identical to each other and share 79.5% sequence identity to SARS-CoV. Furthermore, the SARS-CoV-2 is 96% identical at the whole genome level to a bat coronavirus. Phylogenetic analysis of CoVs of different species indicated that SARS-CoV-2 could have originated from Chinese horseshoe bats, but the intermediate transmission vehicle has not yet been identified (Dong, N. et al. Microbiology 2020). According to this study, SARS-CoV-2 belongs to a novel type of bat coronavirus owing to a high degree of variation from the human SARS virus. SARS-CoV-2 is the seventh member of the family of CoVs that infect humans.

Like SARS-CoV, SARS-CoV-2 enters target cells through an endosomal pathway and also uses the same cell entry receptor, Angiotensin-converting enzyme II (ACE2).

An effective therapeutic targets in SARS-CoV and several other CoVs, are replication-related enzymes, such as proteases (Zhou, P. et al. Microbiology 104, 2020). Drugs that inhibit conserved proteases are capable of preventing replication and proliferation of the virus by interfering with the post-translational processing of essential viral polypeptides. They can also reduce the risk of mutation-mediated drug resistance. This was the case for the SARS-CoV, as inhibitors targeting the main protease involved in replication and proliferation were the most effective means to alleviate the epidemic.

A particularly interesting therapeutic target is 3C-like proteinase (3CLP), a homodimeric cysteine protease and a member of a family of enzymes found in the Coronavirus polyprotein (Fan, K et al., J.Biol.Chem.279,1637-1642 (2004)). 3CLP in CoVs share conserved substrate recognition pockets, which are responsible for cleaving the viral polyprotein and the host factors involved in the innate immune response (Perlman, S et al., Nat. Rev. Microbiol. 7, 439-450). Due to its role in processing and virus maturation, 3CLP is considered to be a target for viral inhibitor development as an approach toward the severe acute respiratory syndrome coronavirus (SARS-Cov) treatment. (Ahn TY et al., Bull Korean Chem Soc. 2010;31(1):87-91) (Liang PH et al., Curr. Top Med Chem. 2006;6(4):361-76) (Anand et al., Science 2003;300(5626):1763-7).

Computational drug repurposing is an effective approach to find new identifications for already known drugs. (Vanhaelen, Q et al., Drug Discov. Today 22, 210-222(2017) (Karman, B&Sippl, Curr.Med.Chem.26, 5389-5409(2019).

### SUMMARY OF THE INVENTION

The present inventors have carried out analysis of a library containing commercialized and under development drugs safe in man (>10000 drugs) using a highly reliable computational screening platform, as described in details in the experimental section, and have identified that a number of known compounds already approved for clinical use in humans, are able to bind and inhibit the activity of the 3C-like protease (RCLP) of SARS-Cov-2 virus. These compounds are therefore useful for the treatment of infections from the above virus.

Accordingly, a first object of the invention is a compound inhibitor of 3CLP of SARS-Cov-2 for use in the treatment of COVID-19, wherein said compound is a compounds of table 1 below.

A second object of the invention is a pharmaceutical composition comprising i) a compound inhibitor of 3CLP of SARS-Cov-2 of table 1 below and ii) at least one inert pharmaceutically acceptable excipient, for use in the treatment of COVID-19.

A third object of the invention is a method of treating COVID-19, comprising administering to a patient affected thereby a compound of table 1 below.

### DETAILED DESCRIPTION OF THE INVENTION

A first object of the present invention is a compound inhibitor of 3CLP of SARS-CoV-2 for use in the treatment of COVID-19, wherein said compound is a compound of Table 1 below:

**Table 1**

| **ID N-** | **Chemical Name** | **Generic Name (GN)** |
|---|---|---|
| 1 | N1-[4-[5-(2-Phenanthryl)-3-(trifluoromethyl)-1H-pyrazol-1-yl]phenyl]glycinamide | |
| 2 | N-(5-[3-[7-(3-Fluorophenyl)-1H-imidazo[4,5-c]pyridin-2-yl]-1 H-indazol-5-yl]pyridin-3-yl)-3-methylbutanamide | Adavivint (former INN); Lorecivivint (USAN; Rec INN) |
| 3 | N-[3-Chloro-4-[(12aS)-3,4,6,11,12,12a-hexahydro-1H-[1,4]oxazino[3,4-c][1,4]benzodiazepin-11-ylcarbonyl]phenyl]biphenyl-2-carboxamide hydrochloride | |
| 4 | 2-(5-[(Z)-[1-(4-tert-Butylphenyl)-5-oxo-3-phenyl-1,5-dihydro-4H-pyrazol-4-ylidene]methyl]furan-2-yl)-4-chlorobenzoic acid | |
| 5 | 2-[1-(4-tert-Butylbenzyl)-5-(3-methylphenyl)-1 H-indol-3-yl]-2-oxoacetic acid | Aleplasinin (Prop INN; USAN) |
| 6 | N'-[4-[4-[2-[3-(Dimethylaminomethyl)phenyl]-1H-pyrrolo[2,3-b]pyridin-4-yl]-1-ethyl-1H-pyrazol-3-yl]phenyl]-N,N-dimethylurea | |
| 7 | (4S,5R)-5-[3,5-Bis(trifluoromethyl)phenyl]-3-([2-[4-fluoro-2-methoxy-5-(propan-2-yl)phenyl]-5,5-dimethylcyclohex-1-en-1-yl]methyl)-4-methyl-1,3-oxazolidin-2-one | Rocacetrapib (Rec INN) |
| 8 | [2-[6-(2-Ethyl-5-fluoro-4-hydroxyphenyl)-1H-indazol-3-yl]-1,4,6,7-tetrahydro-5H-imidazo[4,5-c]pyridin-5-yl][5-(piperidin-1-yl)pyrazin-2-yl]methanone | |
| 9 | 2-Bromoergocryptine monomethanesulfonate | Bromocriptine mesilate (BANM; Rec INNM; USAN; JAN) |
| 10 | (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester; (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydronicotinic acid ethyl ester | Modipafant (BAN; Rec INN) |
| 11 | 4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester | |
| 12 | 4-Methyl-N-[3-(4-methyl-1H-imidazol-1-yl)-5-(trifluoromethyl)phenyl]-3-[4-(3-pyridyl)pyrimidin-2-ylamino]benzamide hydrochloride monohydrate | Nilotinib hydrochloride monohydrate (Rec INNM; USAN) |
| 13 | 4-Chloro-2-[5-[[1-(4-fluorophenyl)-5-oxo-3-phenyl-1,5-dihydro-4H-pyrazol-4-ylidene]methyl]-2-furyl]benzoic acid | |
| 14 | 2-Chloro-6-methyl-N'-[[4-methyl-3-(quinolin-3-ylethynyl)phenyl]carbonyl]benzohydrazide | |
| 15 | (±)-N-(4-Amino-3,4-dioxo-1-phenylbutan-2-yl)-2-[3-(4-fluorophenyl)-1 H-pyrazol-1-yl]pyridine-3-carboxamide | |
| 16 | 3(R)-(2,3-Dihydro-1-benzofuran-5-yl)-2-[5-(2-pyridyl)pyrimidin-2-yl]-2,3,4,9-tetrahydro-1H-pyrrolo[3,4-b]quinolin-9-one | |
| 17 | 4-Chloro-2-[5-[(5-oxo-1,3-diphenyl-1,5-dihydro-4H-pyrazol-4-ylidene)methyl]-2-furyl]benzoic acid | |
| 18 | 4-Amino-N-[1-(3-chloro-2-fluoroanilino)-6-methylisoquinolin-5-yl]thieno[3,2-d]pyrimidine-7-carboxamide | Belvarafenib (Rec INN) |
| 19 | (S,S)-6,6'-[Carbonylbis(5-imino-1 H-indole-2-carbonyl)]bis[8-(chloromethyl)-1-methyl-3,6,7,8-tetrahydrobenzo[1,2-b:4,3-b']dipyrrol-4-ol]; (S,S)-1,3-Bis[2-[1-(Chloromethyl)-5-hydroxy-8-methyl-1,2,3,6-tetrahydrobenzo[1,2-b:4,3-b']dipyrrol-3-ylcarbonyl]-1 H-indol-5-yl]urea | Bizelesin (USAN; Rec INN) |
| 20 | N-[3-[6-[4-[1,4-Dimethyl-3-oxopiperazin-2(R)-yl]phenylamino]-4-methyl-5-oxo-4,5-dihydropyrazin-2-yl]-2-methylphenyl]-4,5,6,7-tetrahydro-1-benzothiophene-2-carboxamide | |
| 21 | 2-Hydroxy-5-[4-[3-[4-(2-methylimidazo[4,5-c]pyridin-1-ylmethyl)piperidin-1-yl]-3-oxo-1-phenyl-1 (Z)-propenyl]phenylazo]benzoic acid | Dersalazine (Rec INN) |
| 22 | (-)-2-[3-(1,4-Dimethyl-1H-1,2,3-triazol-5-yl)-5-[(S)-phenyl(tetrahydro-2H-pyran-4-yl)methyl]-5H-pyrido[3,2-b]indol-7-yl]propan-2-ol | |
| 23 | 4-[(2R,4R)-4-[[1-(2,2-Difluoro-2H-1,3-benzodioxol-5-yl)cyclopropane-1-carbonyl]amino]-7-(difluoromethoxy)-3,4-dihydro-2H-1-benzopyran-2-yl]benzoic acid | Galicaftor (USAN; Rec INN) |
| 24 | 2-[5-[(5-Oxo-1,3-diphenyl-1,5-dihydro-4H-pyrazol-4-yl idene) methyl]furan-2-yl]benzoic acid | |
| 25 | 1-[2-[1-[3,5-Bis(trifluoromethyl)benzyl]-5-(4-pyridyl)-1 H-1,2,3-triazol-4-yl]pyridin-3-yl]-1-(2-chlorophenyl)methanone | Tradipitant (Prop INN; USAN) |
| 26 | 6-[(5S,9R)-9-(4-Cyanophenyl)-3-(3,5-dichlorophenyl)-1-methyl-2,4-dioxo-1,3,7-triazaspiro[4.4]non-7-yl]pyridine-3-carboxylic acid | |
| 27 | (6S)-6-(2-Hydroxy-2-methylpropyl)-3-[(1S)-1-[4-(1 -methyl-2-oxo-1,2-dihydropyridin-4-yl)phenyl]ethyl]-6-phenyltetrahydro-2H-1,3-oxazin-2-one hydrate | |
| 28 | Carbonic acid cholest-5-en-3beta-yl 3,5-dichloro-2-methoxy-6-(trichloromethyl)pyridin-4-yl diester | 4-Demethyl-4-cholesteryloxycarbonylpenc lomedine |
| 29 | 3-[3-[1-(N-Isopropyl-N-phenylcarbamoylmethyl)-2,4-dioxo-5-phenyl-2,3,4,5-tetrahydro-1H-1,5-benzodiazepin-3(S)-yl]ureido]benzoic acid | |
| 30 | 2-[3-[N-Methyl-N-[1-(naphthalen-2-ylcarbonyl)piperidin-4-yl]carbamoyl]naphthalen-2-yl]-1-(1-naphthyl)-2-oxoethylphosphonic acid | |
| 31 | 2-Propyl-1-[2'-(1H-tetrazol-5-yl)biphenyl-4-ylmethyl]-4-[2-(trifluoroacetyl)pyrrol-1-yl]imidazole-5-carboxylic acid | |
| 32 | 4'-[4-Methyl-6-(1-methyl-1 H-benzimidazol-2-yl)-2-propyl-1 H-benzimidazol-1-ylmethyl]biphenyl-2-carboxylic acid | Telmisartan (USAN; Rec INN) |
| 33 | 5-Chloro-N-(3-cyclopropyl-5-[(cis-3,5-dimethylpiperazin-1-yl)methyl]phenyl)-4-(6-methyl-1 H-indol-3-yl)pyrimidin-2-amine hydrate | |
| 34 | 11-Keto-beta-boswellic acid; 3alpha-Hydroxyurs-12-en-11-keto-23-oic acid | 11-Keto-beta-boswellic acid |
| 35 | N-(4-tert-Butylphenyl)-N-[2-[(2-methylpropyl)amino]-2-oxo-1-(pyridin-3-yl)ethyl]-1 H-imidazole-4-carboxamide | |
| 36 | 5-[N-[5-[N-(Cycloheptylmethyl)carbamoyl]-1 H-benzi midazol-6-ylcarbonyl]-2- fluoro-L-phenylalanylamino]isophthalic acid; 5-[N-[5-[N-(Cycloheptylmethyl)carbamoyl]-1 H-benzimidazol-6-ylcarbonyl]-2-fluoro-L-phenylalanylaminolbenzene-1,3-dicarboxylic acid | Gastrazole |
| 37 | 21-[4-[2,6-Bis(pyrrolidino)pyrimidin-4-yl]piperazin-1-yl]-16alpha-methyl-pregna-1,4,9(11)-triene-3,20-dione methanesulfonate hydrate; 16alphaMethyl-21-[4-[2,6-bis(pyrrolidino)-4-pyrimidinyl]-1-piperazinyl]pregna-1,4,9(11)triene-3,20-dione monomethanesulfonate hydrate | Tirilazad mesylate (Rec INNM; BAN; USAN) |
| 38 | 3-[5-Oxo-3-phenyl-4-[(5-phenylfuran-2-yl)methylidene]-4,5-dihydro-1 H-pyrazol-1-yl]benzoic acid | |
| 39 | 2-[2-[2-(2-Chlorophenyl)propan-2-yl]-1-[3'-(methylsulfonyl)biphenyl-4-yl]-1 H-imidazol-4-yl]propan-2-ol | |
| 40 | 4-[4-[3-[3-tert-Butyl-1-(6-quinolinyl)-1H-pyrazol-5-yl]ureido]-3-fluorophenoxy]-N-methylpyridine-2-carboxamide tosylate salt | Rebastinib tosylate (USAN; Rec INN) |
| 41 | Benzoic acid 4-chloro-3-[5-methyl-3-[4-[2-(1-pyrrolidinyl)ethoxy]phenylamino]-1,2,4-benzotriazin-7-yl]phenyl ester | |
| 42 | N-[3-Fluoro-4-[[11 C]methyloxy]phenyl]-6-[(2R)-2-(3-fluorophenyl)pyrrolidin-1-yl]imidazo[1,2-b]pyridazine-3-carboxamide | |
| 43 | 4-Fluoro-2-(4-fluorophenyl)-N-methyl-5-(2-methyl-5-[[1-(pyrimidin-2-yl)cyclopropyl]carbamoyl]phenyl)-1-benzofuran-3-carboxamide | |
| 44 | 6-Methyl-N5-[3-(1 H-purin-6-yl)pyridin-2-yl]-N1-[3-(trifluoromethoxy)phenyl]isoquinoline-1,5-diamine | |
| 45 | (aR)-6-Fluoro-7-(2-fluoro-6-hydroxyphenyl)-1-[4-methyl-2-(propan-2-yl)pyridin-3-yl]-4-[(2S)-2-methyl-4-(prop-2-enoyl)piperazin-1-yl]pyrido[2,3-d]pyrimidin-2(1H)-one | |
| 46 | N-(3-Fluoro-4-[[7-(2-hydroxy-2-methylpropoxy)quinolin-4-yl]oxy]phenyl)-1,5-dimethyl-3-oxo-2-phenyl-2,3-dihydro-1 H-pyrazole-4-carboxamide 4-methylbenzenesulfonate | Ningetinib Tosylate; Ningetinib (free base) |
| 47 | 4-[2-[4-(4-Ethylphenyl)-2,2-dimethyl-2H-1-benzothiopyran-6-yl]ethynyl]benzoic acid | |
| 48 | (2E)-3-(6-Aminopyridin-3-yl)-N-([5-[4-(4,4-difluoropiperidine-1-carbonyl)phenyl]-7-(4-fluorophenyl)-1-benzofuran-2-yl]methyl)prop-2-enamide | |
| 49 | 3-Chloro-N-[1-(N,N-dimethylglycinamido)-3-[4-[8-[1(S)-hydroxyethyl]imidazo[1,2-a]pyridin-2-yl]phenyl]propan-2(S)-yl]-4-isopropoxybenzamide | |
| 50 | Phosphoric acid (6aR,9aS)-3-anilino-2-[[4-(6-fluoropyridin-2-yl)phenyl]methyl]-5-methyl-5,6a,7,8,9,9a-hexahydrocyclopenta[4,5]imidazo[1,2-a]pyrazolo[4,3-e]pyrimidin-4(2H)-one | |
| 51 | (4R)-1-[[3-Acetyl-5-(2-methylpyrimidin-5-yl)-1 H-indazol-1-yl]acetyl]-N-(6-bromopyridin-2-yl)-4-fluoro-L-prolinamide | Danicopan (USAN; Rec INN) |
| 52 | 1-[3-Bromo-2-[2-(trifluoromethylsulfonamido)phenyl]benzofuran-5-ylmethyl]-4-cyclopropyl-2-ethyl-1 H-imidazole-5-carboxamide | Saprisartan (BAN; USAN; Rec INN) |
| 53 | 2-(4-Fluorophenyl)-5-(11-fluoro-6H-pyrido[2',3':5,6][1,3]oxazino[3,4-a]indol-2-yl)-6-[(methanesulfonyl)(methyl)aminol-N-methyl-1-benzofuran-3-carboxamide | |
| 54 | Bis(4,4'-dimethyl-2,2'-bipyridine) 2-[[1(2),2(2):2(5),3(2)-terthiophen]-1(5)-yl]-1 H-imidazo[4,5-f][1,10]phenanthroline dichloridoruthenium | |
| 55 | N-[4-[2-[4-[3-Ethoxy-1 (E)-propenyl]phenyl]-4-[4-(isopropylamino)phenyl]-1 H-imidazol-5-yl]phenyl]-N-isopropylamine; 2-[4-[3-Ethoxy-1(E)-propenyl]phenyl]-4,5-bis[4-(isopropylamino)phenyl]-1 H-imidazole | |
| 56 | Phosphoric acid [(3beta,5alpha,24R)-stigmastan-3-yl] L-ascorbic acid-2-O-yl diester disodium salt; 2-O-[Hydroxy[(3beta,5alpha,24R)-stigmastan-3-O-yl]phosphoryl]-L-ascorbic acid disodium salt | Sitostanol ascorbyl phosphate |
| 57 | N-[4-Methyl-3-[4-(3-pyridyl)pyrimidin-2-ylamino)phenyl]-3,5-bis(trifluoromethyl)benzamide | |
| 58 | 1-(6,7-Dihydro-5H-benzo[6,7]cyclohepta[1,2-c]pyridazin-3-yl)-N3-[7(S)-(1-pyrrolidinyl)-6,7,8,9-tetrahydro-5H-benzocycloheptene-2-yl]-1 H-1,2,4-triazole-3,5-diamine | Bemcentinib (USAN; Rec INN) |
| 59 | 5-[5-Phenyl-4-(pyridin-2-ylmethylamino)quinazolin-2-yl]pyridine-3-sulfonamide | |
| 60 | N-[2-(2,1,3-Benzothiadiazol-5-ylamino)-6-(2,6-dichlorophenyl)pyrido[2,3-d]pyrimidin-7-yl]-N'-tert-butylurea | |
| 61 | N-[4-(2-Methyl-1,4,5,6-tetrahydroimidazo[4,5-d][1]benzazepin-6-ylcarbonyl)phenyl]biphenyl-2-carboxamide hydrochloride | Conivaptan hydrochloride (Rec INNM) |
| 62 | N-[4-[3-(N-Benzyl-N-methylaminomethyl)-7-(2,6-difluorobenzyl)-5-isobutyryl-4-oxo-4,7-dihydrothieno[2,3-b]pyridin-2-yl]phenyl]-1-hydroxycyclopropanecarboxamide | |
| 63 | 2-(3-Chloro-4-fluorobenzyl)-8-ethyl-10-hydroxy-6(S)-methyl-4-(5-methyl-1,1-dioxido-1,2,5-thiadiazolidin-2-yl)-1,2,6,7,8,9-hexahydropyrazino[1',2':1,5]pyrrolo[2,3-d]pyridazine-1,9-dione | |
| 64 | (7bR,8aS)-N-[2-[(1,2,4,5,8,8a-Hexahydro-7-methyl-4-oxocyclopropa[c]pyrrolo[3,2-e]indol-2-yl)carbonyl]-1 H-indol-5-yl]-2-benzofurancarboxamide | Adozelesin (USAN; Rec INN) |
| 65 | N-(2,4-Di-tert-butyl-5-hydroxyphenyl)-4-oxo-1,4-dihydroquinoline-3-carboxamide | Ivacaftor (Prop INN; USAN) |
| 66 | N-(4-tert-Butylphenyl)-N-[2-(cyclohexylamino)-2-oxo-1-(pyridin-3-yl)ethyl]-1 H-imidazole-4-carboxamide | |
| 67 | 1-(3,4-Dimethylphenyl)-3-methyl-4,5-dihydro-1 H-pyrazole-4,5-dione 4-[4-[2-hydroxy-3'-(1 H-tetrazol-5-yl)biphenyl-3-yl]hydrazone choline salt | Totrombopag choline (Prop INNM; USAN) |
| 68 | Piperazine-1,4-diylbis[[6-(1H-benzimidazol-2-yl)pyridin-2-yl]methanone] | |
| 69 | 4,5,6,7-Tetrachloro-2',4',5',7'-tetraiodo-3-oxo-3H-spiro[2-benzofuran-1,9'-xanthene]-3',6'-diolate disodium salt | Rose Bengal disodium |
| 70 | 3-(4-Chlorophenylamino)-10-(4-chlorophenyl)-2,10-dihydro-2-(isopropylimino)phenazine | Clofazimina; Clofazimine (BAN; USAN; Rec INN) |
| 71 | (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide | Avacopan (USAN; Rec INN) |
| 72 | 4-[[(3S,4R,5S)-3-(3-Chloro-2-fluorophenyl)-4-(4-chloro-2-fluorophenyl)-4-cyano-5-(2,2-dimethylpropyl)-D-prolyl]amino]-3-methoxybenzoic acid | Idasanutlin (USAN; Rec INN) |
| 73 | 1-(2-Naphthylmethyl)-3-[(4-nitrophenyl)imino]-2-oxoindoline-5-carboxamide | |
| 74 | N-[3-(7-Methyl-1H-indazol-5-yl)-1-[4-(1-methylpiperidin-4-yl)piperazin-1-yl]-1-oxopropan-2(R)-yl]-4-(2-oxo-1,2-dihydroquinolin-3-yl)piperidine-1-carboxamide | Vazegepant |
| 75 | 2-[2-Fluoro-4-[7-(2-methylpyridin-4-yl)imidazo[1,2-a]pyridin-3-yl]phenyl]-N-[3-(trifluoromethyl)phenyl]acetamide | |
| 76 | N-(2-Fluoro-6-methylphenyl)-6-(4-[[5-methyl-2-(7-oxa-2-azaspiro[3.5]nonan-2-yl)pyridine-3-carbonyl]amino]benzoyl)-5,6-dihydro-4H-thieno[3,2-d][1]benzazepine-2-carboxamide | |
| 77 | 2-[2-[2-[2(E)-[4-(Trifluoromethyl)phenyl]vinyl]-1 H-benzimidazol-5-yl]phenyl]propan-2-ol | Mavatrep (USAN; Rec INN) |
| 78 | 4-Methyl-N-[3-(4-methyl-1H-imidazol-1-yl)-5-(trifluoromethyl)phenyl]-3-[4-(2-pyrazinyl)pyrimidin-2-ylamino]benzamide | Radotinib (Prop INN) |
| 79 | 7-(4,7-Diazaspiro[2.5]octan-7-yl)-2-(2,8-dimethylimidazo[1,2-b]pyridazin-6-yl)-4H-pyrido[1,2-a]pyrimidin-4-one | Risdiplam (USAN; Rec INN) |
| 80 | 3-[(4-Methylphenyl)imino]-1-(2-naphthylmethyl)-2-oxoindoline-5-carboxamide | |
| 81 | 4-(4-Methylpiperazin-1-ylmethyl)-N-[6-methyl-5-[4-(3-pyridyl)pyrimidin-2-ylamino]pyridin-3-yl]-3-(trifluoromethyl)benzamide methanesulfonate | Flumatinib mesylate |
| 82 | 2-(5-Amino-6-oxo-2-phenyl-1,6-dihydropyrimidin-1-yl)-N-[1-(5-tert-butyl-1,3,4-oxadiazol-2-ycarbonyl)-2-methylpropyl]acetamide | Freselestat (Rec INN) |
| 83 | N-[3-[5-(2-Aminopyrimidin-4-yl)-2-tert-butylthiazol-4-yl]-2-fluorophenyl]-2,6-difluorobenzenesulfonamide methanesulfonate | Dabrafenib mesylate (Prop INNM; USAN) |
| 84 | (OC-6-23)-[[3-(Dimethylamino)propyl]dimethylsilinolatokappaO]hydroxy[29H,31H-phthalocyanato(2-)-kappaN29,kappaN30,kappaN31,kappaN32]silico n | Silicon phthalocyanine 4 |
| 85 | Methanesulfonic acid 2-[3-[4-[(1 H-indazol-5-yl)amino]quinazolin-2-yl]phenoxy]-N-(propan-2-yl)acetamide | |
| 86 | Sodium (2E)-3-[2-[1-[[[2-(5-bromopyrimidin-2-yl)-3-cyclopentyl-1-methyl-1H-indol-6-yl]carbonyl]amino]cyclobutyl]-1-methyl-1H-benzimidazol-6-yl]acrylate | Deleobuvir sodium (USAN; Rec INN) |
| 87 | [4(R),6(S),(E)]-6-[2-[4-(4-Fluorophenyl)-2-isopropyl-6-phenyl-3-pyridyl]vinyl]-4-hydroxytetrahydropyran-2-one | Glenvastatin (Rec INN) |
| 88 | [1-(4-[[4-(4-Hydroxypiperidin-1-yl)phenyl]amino]-5-oxo-5,6-dihydropyrimido[4,5-d]pyridazin-2-yl)piperidin-4-yl]acetonitrile | Gusacitinib (Prop INN; USAN) |
| 89 | N-[2-(Benzylamino)-2-oxo-1-(pyridin-3-yl)ethyl]-N-(4-tert-butylphenyl)-1H-imidazole-4-carboxamide | |
| 90 | N6-(4,4-Dimethyl-4,5-dihydroisoxazol-2-yl)-N4-[3-methyl-4-([1,2,4]triazolo[1,5-a]pyridin-7-yloxy)phenyl]quinazoline-4,6-diamine | Irbinitinib (former INN); Tucatinib (Prop INN; USAN) |
| 91 | 5-Chloro-N2-[5-methyl-4-(piperidin-4-yl)-2-(propan-2-yloxy)phenyl]-N4-[2-(propan-2-ylsulfonyl)phenyl]pyrimidine-2,4-diamine | Ceritinib (USAN; Rec INN) |
| 92 | (2S)-tert-Butoxy[4-(2,3-dihydropyrano[4,3,2-de]quinolin-7-yl)-2-methylquinolin-3-yl]ethanoic acid | |
| 93 | cis-3-[8-Amino-1-(2-phenylquinolin-7-yl)imidazo[1,5-a]pyrazin-3-yl]-1-methylcyclobutanol | Linsitinib (Prop INN; USAN) |
| 94 | (Z)-2-[4-[2-[1-[4-[(Propan-2-yl)oxy]phenyl]-1 H-benzimidazol-5-yl]ethenyl]phenyl]propan-2-ol | |
| 95 | 6alpha,9alpha-Difluoro-16alpha,17alpha-[4-(dimethylamino)phenylmethylenedioxy]-11beta-hydroxy-21-(3-benzamido-2-methylpropionyloxy)pregna-1,4-dien-3-one | |
| 96 | N-Cyclopropyl-4-[7-[[(cis-3-hydroxy-3-methylcyclobutyl)methyl]amino]-5-(pyridin-3-yloxy)pyrazolo[1,5-a]pyrimidin-3-yl]-2-methylbenzamide hydrochloride | |
| 97 | (+)-(R)-1-[4-(2-Chloro-5-fluorobenzamido)-3-methoxybenzoyl]-1,2,3,5-tetrahydrospiro[1-benzazepine-4,1'-[2]cyclopentene]-3'-carboxylic acid | |
| 98 | 3-[4-[1-[3-Cyclopentyl-1-methyl-2-(2-pyridyl)-1 H-indol-6-ylcarboxamido]cyclobutylcarboxamido]phenyl]-2-propenoic acid trifluoroacetate | |
| 99 | 6-(1 H-Indazol-6-yl)-N-[4-(morpholin-4-yl)phenyl]imidazo[1,2-a]pyrazin-8-amine di methanesulfonate | Entospletinib dimesylate (Rec INN) |
| 100 | 2-[(1S)-1-[4-Amino-3-(3-fluoro-4-isopropoxyphenyl)-1H-pyrazolo[3,4-d]pyrimidin-1-yl]ethyl]-6-fluoro-3-(3-fluorophenyl)-4H-chromen-4-one 4-methylbenzenesulfonate | Umbralisib tosylate (Rec INNM; USAN) |
| 101 | 2-Butyl-5-[[11C]-methoxymethyl]-6-(1-oxidopyridin-2-yl)-3-[2'-(1 H-tetrazol-5-yl)biphenyl-4-ylmethyl]-3H-imidazo[4,5-b]pyridine | |
| 102 | 3-Amino-3-methyl-N-[2-oxo-1-[2'-(1 H-tetrazol-5-yl)biphenyl-4-ylmethyl]-2,3,4,5-tetrahydro-1 H-1- benzazepin-3(R)-yl]butyramide | |
| 103 | 4-([[(1S)-2-[(2E)-3-[3-Chloro-2-fluoro-6-(1 H-tetrazol-1-yl)phenyl]prop-2-enoyl]-5-(4-methyl-2-oxopiperazin-1-yl)-1,2,3,4-tetrahydroisoquinolin-1-yl]carbonyl]amino)benzoic acid hydrochloride | |
| 104 | 5-[2-Hydroxy-3-[(2Z)-2-[3-methyl-5-oxo-1-(5,6,7,8-tetrahydronaphthalen-2-yl)-4,5-dihydro-1 H-pyrazol-4-ylidene]hydrazino]phenyl]furan-2-carboxylic acid bis(2-aminoethanol) | Hetrombopag olamine |
| 105 | 3-[4-(1-Cyclobutylpiperidin-4-yloxy)phenyl]-2-methyl-5-(trifluoromethyl)quinazolin-4(3H)-one | |
| 106 | (R,S)-9-[Benzyloxy(hydroxy)phosphoryloxy]-14-methyl-5,14-dihydrobenz[5,6]isoindolo[2,1-b]isoquinoline-8,13-dione; Benzyl (±)-5,8,13,14-tetrahydro-14-methyl-8,13-dioxobenz[5,6]isoindolo[2,1-b]isoquinolin-9-yl hydrogen phosphate | Fosquidone (BAN; USAN; Rec INN) |
| 107 | 5-[6-[2,4-Bis(trifluoromethyl)phenyl]pyridazin-3-ylmethyl]-2-(2-fluorophenyl)-5H-imidazo[4,5-c]pyridine | Tegobuvir (Prop INN; USAN) |
| 108 | 2,7-Bis[[(3R,5S)-3,5-dimethylpiperidin-1-yl]sulfonyl]-N,N'-dihydroxyanthracene-9,10-diimine | Tegatrabetan; Tegavivint (Rec INN) |
| 109 | 4'-[2-Ethyl-4-methyl-6-(5,6,7,8-tetrahydroimidazo[1,2-a]pyridin-2-yl)benzimidazol-1-ylmethyl]biphenyl-2-carboxylic acid | Pomisartan (Prop INN) |
| 110 | (6S)-N-[(3S,5S,6R)-6-Methyl-2-oxo-1-(2,2,2-trifluoroethyl)-5-(2,3,6-trifluorophenyl)piperidin-3-yl]-2'-oxo-1',2',5,7-tetrahydrospiro[cyclopenta[b]pyridine-6,3'-pyrrolo[2,3-b]pyridine]-3-carboxamide | Atogepant (Rec INNM; Rec INN) |
| 111 | 5-(4-Cyclopropyl-1 H-imidazol-1-yl)-2-fluoro-N-[6-(4-isopropyl-4H-1,2,4-triazol-3-yl)pyridin-2-yl]-4-methylbenzamide hydrochloride | Selonsertib hydrochloride (USAN; Rec INN) |
| 112 | N-(2-Bromo-3-oxospiro[3.5]non-1-en-1-yl)-4-(2,7-naphthyridin-1-ylamino)-L-phenylalanine ethyl ester | Zaurategrast (Prop INN) |
| 113 | (6aR,9R,10aR)-N-[(2R,5S,10aS,10bS)-10b-Hyd roxy-5- i so butyl-2- iso propyl-3, 6-dioxoperhydrooxazolo[3,2-a]pyrrolo[1,2-d]pyrazin-2-yl]-7-methyl-4,6,6a,7,8,9,10,10a-octahydroindolo[4,3-fg]quinoline-9-carboxamide mesylate | Dihydro-alpha-ergokryptine mesylate |
| 114 | 4-[9-Chloro-7-(2,6-difluorophenyl)-5H-pyrimido[5,4-d][2]benzazepin-2-ylamino]benzoic acid | |
| 115 | 4-[4-[4-[4-Fluoro-3-(trifluoromethyl)phenyl]-1-methyl-1H-imidazol-2-yl]piperidin-1-yl]-1H-pyrazolo[3,4-d]pyrimidine 4-methylbenzenesulfonate | |
| 116 | Lup-20(29)-ene-3beta,28-diol | Betulin; Betulinic alcohol; Betulinol; Trochol |
| 117 | 1-(2-Naphthylmethyl)-2-oxo-3-(phenylimino)indoline-5-carboxamide | |
| 118 | 3,3'-[(16-Dioxo-1,6-hexanediyl)diimino]bis[2,4,6-triiodobenzoic acid] diethyl ester | Iodipamide ethyl ester |
| 119 | 2-(Piperidin-1-yl)-N-[4-(trifluoromethyl)phenyl]-7-[3-(trifluoromethyl)pyridin-2-yl]-6,7,8,9-tetrahydro-5H-pyrimido[4,5-d]azepin-4-amine sulfate | |
| 120 | 4,5-Dichloro-N-[3-[1-(2,4-dichlorobenzyl)-5-fluoro-3-methyl-1 H-indol-7-yl]-2(E)-propenoyl]thiophene-2-sulfonamide | |
| 121 | (2E)-N-(4-[[3-Chloro-4-(pyridin-2-ylmethoxy)phenyl]amino]-3-cyano-7-ethoxyquinolin-6-yl)-3-[(2R)-1-methylpyrrolidin-2-yl]prop-2-enamide (2Z)-but-2-enedioate | Pyrotinib maleate |
| 122 | (2S)-N-[(4S,6S,7S)-7-[[(2,6-Dimethylphenoxy)acetyl]amino]-6-hydroxy-2-methyl-8-phenyloctan-4-yl]-3-methyl-2-(2-oxotetrahydropyrimidin-1(2H)-yl)butanamide | Leucine lopinavir |
| 123 | 4-[2,6-Di-tert-butyl-4-[1-(3,5-di-tert-butyl-4-hydroxyphenylsulfanyl)-1-methylethylsulfanyl]phenoxy]butyric acid | Elsibucol (USAN; Rec INN) |
| 124 | N-[2-(1H-1,2,4-Triazol-5-ylmethyl)-1,2,3,4-tetrahydroisoquinolin-6-yl]-4'-(trifluoromethyl)-1,1'-biphenyl-2-carboxamide | |
| 125 | (1S)-1-(4-Fluorophenyl)-1-(2-[4-[6-(1-methyl-1H-pyrazol-4-yl)pyrrolo[2,1-f][1,2,4]triazin-4-yl]piperazin-1-yl]pyrimidin-5-yl)ethan-1-amine | Avapritinib (Rec INNM; Rec INN) |
| 126 | 2-Hydroxy-N,N,N-trimethylethan-1-aminium (3beta,18alpha)-3-hydroxy-11-oxoolean-12-en-30-oate | 18alpha-Glycyrrhetinic acid choline salt |
| 127 | N-[4-[3-(2-Aminopyrimidin-4-yl)pyridin-2-yloxy]phenyl]-4-(4-methylthien-2-yl)phthalazin-1-amine | |
| 128 | 2-Hydroxy-1-[2-[[9-(trans-4-methylcyclohexyl)-9H-pyrido[4',3':4,5]pyrrolo[2,3-d]pyrimidin-2-yl]amino]-7,8-dihydro-1,6-naphthyridin-6(5H)-yl]ethanone | |
| 129 | 9-Isopropyl-N2-[4-(4-methylpiperazin-1- yl)phenyl]-N8-(3-pyridyl)-9H-purine-2,8-diamine | |
| 130 | 1(RS)-N-[4-[N-(4-Chlorophenylsulfonyl)carbamoyl]benzoyl]-L-valyl-N-[2-methyl-1-(trifluoroacetyl)propyl]-L-prolinamide | |
| 131 | 2-Amino-5-cyano-6-methyl-4(R)-(3-phenylquinolin-5-yl)-1,4-dihydropyridine-3-carboxylic acid isopropyl ester | |
| 132 | N-[3-tert-Butyl-1-(4-methylphenyl)-1H-pyrazol-5-yl]-N'-[4-[6-(morpholin-4-ylmethyl)pyridin-3-yl]naphthalen-1-yl]urea | |
| 133 | 5-Fluoro-N-[2-[1-methylpyrrolidin-2(R)-yl]ethyl]-3-oxo-6-[3-(2-pyrazinyl)pyrrolidin-1-yl]-3H-benzo[b]pyrido[3,2,1-kl]phenoxazine-2-carboxamide | Itarnafloxin (Prop INN); Quarfloxin (former INN; USAN) |
| 134 | (2R,4aS,6aS,12bR,14aS,14bR)-10-Hydroxy-2,4a,6a,9,12b,14a-hexamethyl-11-oxo-1,2,3,4,4a,5,6,6a,11,12b,13,14,14a,14b-tetradecahydropicene-2-carboxylic acid L-threonine cocrystal | Tripterine threonine |
| 135 | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate | Nelfinavir mesilate (Prop INNM; USAN) |
| 136 | 9-Acetoxy-2-(1,2-dimethylpropyl)-8-(hexopyranosyloxy)-2,4a,7,7,10a,12a-hexamethyl-1,2,3,4,4a,5,6,6a,7,8,9,10,10a,11,12,12a-hexadecahydrochrysene-1-carboxylic acid | |
| 137 | (+)-2-Amino-N-[(1S)-1-[8-[(1-methyl-1 H-pyrazol-4-yl)ethynyl]-1-oxo-2-phenyl-1,2-dihydroisoquinolin-3-yl]ethyl]pyrazolo[1,5-a]pyrimidine-3-carboxamide | |
| 138 | 4"'-O-Methyldigoxin; (3beta,5beta,12beta,14beta)-[O-(2,6-Dideoxy-4-O-methyl-D-ribo-hexopyranosyl)-(1--4)-O-(2,6-dideoxy-beta-D-ribo-hexopyranosyl)-(1--4)-2,6-dideoxy-D-ribo-hexopyranosyloxy]-12,14-dihydroxy-card-20(22)-enolide | Medigoxin (BAN); Metildigoxin (USAN; JAN; Rec INN) |
| 139 | N-[2-Fluoro-5-[3-[2-(2-pyridyl)vinyl]-1H-indazol-6-ylamino]phenyl]-1,3-dimethyl-1 H-pyrazole-5-carboxamide | |
| 140 | N-(4-tert-Butylphenyl)-N-[2-(cyclopentylamino)-2-oxo-1-(pyridin-3-yl)ethyl]-1 H-imidazole-4-carboxamide | |
| 141 | 7-[7-[(3bR,4aS)-3-Methyl-8-oxo-4,4a,5,8-tetrahydrocyclopropa[c]pyrrolo[3,2-e]indol-6(1H)-ylcarbonyl]-4-hydroxy-5-methoxy-1,2,3,6-tetrahydropyrrolo[3,2-e]indol-3-ylcarbonyl]-4-hydroxy-5-methoxy-1,2,3,6-tetrahydropyrrolo[3,2-e]indole-3-carboxamide | Rachelmycin |
| 142 | (6bS,8aS,11R,12aR,12bS,14aR)-3-Hydroxy-4,6b,8a, 11, 12b, 14a-hexamethyl-2,6b,7,8,8a,9,10,11,12,12a,12b,13,14,14a-tetradecahydropicene-2,10-dione | Maytenin; Tingenin A; Tingenone |
| 143 | (aR)-(2S)-6-Fluoro-5-[3-[(aS)-8-fluoro-1-methyl-2,4-dioxo-1,4-dihydroquinazolin-3(2H)-yl]-2-methylphenyl]-2-(2-hydroxypropan-2-yl)-2,3,4,9-tetrahydro-1H-carbazole-8-carboxamide | |
| 144 | 2-Benzyl-5-(4-chlorophenyl)-6-[4-(methylsulfanyl)phenyl]pyridazin-3(2H)-one | |
| 145 | 3-[3-Carboxy-2-methyl-1-[(Z)-5,5,8,8-tetramethyl-5,6,7,8-tetrahydroanthracen-2-ylmethylene]-2(Z)-propenyl]benzoic acid; 4-(3-Carboxyphenyl)-3-methyl-5-(5,5,8,8-tetramethyl-5,6,7,8-tetrahydroanthracen-2-yl)-2(Z),4(Z)-pentadienoic acid | |
| 146 | (S)-N-[1-(2-Fluorophenyl)-4-oxo-3,4,6,7-tetrahydropyrrolo[3,2,1-jk][1,4]benzodiazepin-3-yl]-1H-indole-2-carboxamide | Pranazepide (Prop INN) |
| 147 | 2',6-Difluoro-5'-[3-(1-hydroxy-1-methylethyl)imidazo[1,2-b][1,2,4]triazin-7-yl]biphenyl-2-carbonitrile | |
| 148 | 4-(Dimethylamino)-N-[3-[4-[2-hydroxy-1(S)-phenylethylamino]-6-phenylfuro[2,3-d]pyrimidin-5-yl]phenyl]-2-butenamide | |
| 149 | N'-[4-[4-(Dimethylamino)piperidin-1-ylcarbonyl]phenyl]-N-[4-[4,6-di(4-morpholinyl)-1,3,5-triazin-2-yl]phenyl]urea | Gedatolisib (USAN; Rec INN) |
| 150 | 6-(2-Chlorophenyl)-9-[(4-methoxyphenyl)carbamoyl]-1-methyl-4,7,8,10-tetrahydropyrido[4',3':4,5]thieno[3,2-f][1,2,4]triazolo[4,3-a][1,4]diazepine | Setipafant (Rec INN) |
| 151 | N-(1',2-Dihydroxy-1,2'-binaphthalen-4'-yl)-4-methoxybenzenesulfonamide | |
| 152 | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid 2-aminoethanol (1:2) | Eltrombopag olamine (Rec INNM; USAN) |
| 153 | (11beta,16beta)-9-Fluoro-1',4'-dihydro-11,21-dihydroxy-2'H-naphtho[2',3':16,17]pregna-1,4-diene-3,20-dione monohydrate; 9-Fluoro-1',4'-dihydro-11beta,21-dihydroxy-2'betaH-naphtho[2',3':16,17]pregna-1,4-diene-3,20-dione monohydrate | Naflocort (BAN; Rec INN) |
| 154 | N-(3-[5-[(1-Ethylpiperidin-4-yl)(methyl)amino]-3-(pyrimidin-5-yl)-1H-pyrrolo[3,2-b]pyridin-1-yl]-2,4-difluorophenyl)propane-1-sulfonamide | |
| 155 | N-Cycloheptyl-N-(9H-fluoren-2-ylmethyl)-N'-(2,4,6-trimethylphenyl)urea | |
| 156 | 5-[(1 R,3R)-1-(1-Ethyl-1H-pyrazol-4-yl)-3-[4-(5-fluoropyridin-2-yl)-1 H-imidazol-2-yl]-2,3,4,9-tetrahydro-1 H-beta-carbolin-1-yl]-3-methyl-1 ,3,4-oxadiazol-2(3H)-one | |
| 157 | N-(5-Ethyl-2,4-dimethylphenyl)-4-[3-fluoro-4-(4-methyl-1 H-imidazol-1-yl)phenyl]thiazol-2-amine | |
| 158 | N-[2-(Diethylamino)ethyl]-2,4-dimethyl-5-[2-oxo-5-(2-phenylthiazol-4-yl)-2,3-dihydro-1 H-indol-3-ylidenemethyl]-1 H-pyrrole-3-carboxamide | Amcasertib (USAN; Rec INN) |
| 159 | N-(4-Oxo-3,4-dihydroquinazolin-2-yl)-L-prolyl-N-methyl-L-3-(2-naphthyl)alanine N-benzyl-N-methylamide | |
| 160 | (5S,6R,8R)-3'-(Methylpolyehtyleneglycol)-5-methyl-7,8,14,15-tetrahydro-2'H,4'H,13H-spiro[5,8-epoxy-4b,8a,14-triazadibenzo[b,h]cycloocta[1,2,3,4-jkl]cyclopenta[e]-as-indacene-6,5'-[1,3]oxazolidine]-2',4', 13-trione | Pegcantratinib (USAN; Rec INN) |

The compounds of table 1 are well-known compounds for which various mechanisms of actions has been described, as detailed in the experimental section.

Some of the above compounds, in particuar compounds 4, 13, 17, 24, 66, 73, 80, 89, 117, 140, 142 are already known as inhibitors of 3CLP of SARS-CoV responsible for the SARS outbreak of 2003. However, while related, the SARS-CoV and SARS-CoV2 viruses are different.

As will be described in details in the experimental section, the present inventors have found that, in addition to their known activity, all these diverse molecules share the ability to binding and inhibiting the 3CLP of SARS-Cov-2, which is essential for the replication of the virus once this has infected a subject.

Threfore, the compounds are able to effectively treat COVID-19.

The selected compounds have the advantage that they have already been approved for clinical use in humans, in some cases are approved pharmaceutical products on the market, and can therefore provide a rapid to implement therapeutic approach to COVID-19.

The compound according to the first object of the invention is administered in form of a pharmaceutical composition formed by admixture of the compound admixed with one or more pharmaceutically acceptable excipients.

Thus, a second object of the invention is a pharmaceutical composition comprising a compound inhibitor of 3CLP of SARS-Cov-2 of table 1 and at least one inert pharmaceutically acceptable excipient, for use in the treatment of COVID-19.

The route of administration of the compound or pharmaceutical composition of the present invention depends on the specific compound used or contained in the pharmaceutical composition and is in accordance with known methods for administration, which can be, for example inhalation, injection or infusion by intravenous, parenterally, topically, rectally, nasally, buccally, vaginally or via an implanted reservoir, or by sustained release systems. The term parenteral as used herein includes intravenous, intraperitoneal, intracerebral, intrathecal, intracranial, intramuscular, intraarticular, intrasynovial, intrasternal, intraocular, intraarterial, subcutaneous, intracutaneous or intralesional injection or infusion techniques.

In one preferred embodiment, the invention provides for administration of the compound or the pharmaceutical composition of the invention such that a sufficient concentration of the compound is reached in the respiratory tract.

Preferably, this is obtained by direct administration of the compound or pharmaceutical composition of the present invention to the respiratory tract. The direct administration to the respiratory tract may be the sole route of administration of the compound or composition or may be combined with administration of the compaounsd or composition via other systemic routes,.

For direct administration of the compound or composition of the invention, a wide variety of devices that are designed for the direct delivery of pharmaceutical compositions and therapeutic formulations to the respiratory tract, may be used. In one aspect of the present invention, the compound or pharmaceutical composition of the present invention is administered in aerosolized or inhaled form.

The pharmaceutical composition of the present invention for direct administration to the respiratory tract as described above, can be in form of a aerosol formulation, as a dry powder or a solution or suspension with a diluent.

Preferably, the pharmaceutically acceptable excipient includes any and all solvents, diluents, or other vehicle, dispersion or suspension aids, surface active agents, isotonic agents, thickening or emulsifying agents, preservatives, solid binders, lubricants and the like, as suited to the particular dosage form desired.

Some examples of materials which can serve as pharmaceutically acceptable excipient include, but are not limited to, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatine; talc; excipients such as cocoa butter and suppository waxes; oils such as peanut oil, cottonseed oil; safflower oil; sesame oil; olive oil; corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; sterilized water; Ringer's solution; buffered saline; dextrose solution; maltodextrin solution; ethyl alcohol; and phosphate buffer solutions. Moreover, the composition of the present invention may be formulated into inhalable or injectable dosage forms such as solutions, suspensions, and emulsions by further adding diluents, dispersants, and surfactants.

Further, the composition of the present invention may be suitably formulated using appropriate methods known in the art or by the method disclosed in Remington's Pharmaceutical Science (recent edition), Mack Publishing Company, Easton Pa.

The terms "pharmaceutically acceptable" and "physiologically acceptable" are intended to define, without any particular limitation, any material suitable for preparing a pharmaceutical composition to be administered to a living being.

The dosage forms can also contain other traditional ingredients such as: preservatives, stabilizers, surfactants, buffers, salts for regulating osmotic pressure, emulsifiers, sweeteners, colorants, flavourings and the like.

The amount of said compound in the pharmaceutical composition of the present invention is the the amount usually employed for other indications of the specific compounds.

The amount can vary over a wide range depending on known factors, for example, the molecule used, the severity of the disease, the patient's body weight, the dosage form, the chosen route of administration, and the number of administrations per day. However, a person skilled in the art can determine the optimum amount in easily and routinely manner.

Also specific dosage and treatment regimens for any particular patient will depend upon a variety of factors, including for example the activity and half life of the specific compound employed, the age, body weight, general health status, sex, diet, severity and course of the disease.

The dosage forms of the pharmaceutical composition of the present invention can be prepared by techniques that are familiar to a pharmaceutical chemist, and comprise mixing, granulation, compression, dissolution, sterilization and the like.

A third object of the invention is a method of treating COVID-19, comprising administering to a patient affected thereby a compound of table 1.

The invention will be further describedin the following examples, which do not limit the scope of the invention described in the claims.

### EXPERIMENTAL PART

### Materials and Methods

The sequence and crystal structure of the 3C-like protease P of SARS-CoV-2, was obtained from the Protein Data Bank with the code 6LU7 (https://www.rcsb.org/structure/6lu7).

The EXSCALATE platform, the most powerful computing resources currently based in Europe to empower smart in-silico drug design (co-funded by the H2020-FET-HPC ANTAREX project), was exploited to perform molecular dynamics (MD) and docking simulations, with the final aim to select and make available molecules active against the 3C-like protease of SARS-CoV-2. Molecular dynamics simulations on the 3C-like protease of SARS-CoV-2 were performed to explore the conformational space of the active site of the protease, and select several protein conformations particularly suitable for docking simulations.

In the first step, MD simulations were carried out on the structure of the 3C-like protease of SARS-CoV-2, prepared ad hoc in order to optimize the 3D structure from a chemical and conformational point of view. The structure was firstly subjected to a cycle of energy minimization by steepest descent methods to eliminate all initial steric clashes and obtain a pre-equilibrated model to start from. Then a 100 ps restrained MD simulation (typically 1000 KJ/mole force constant) was performed on the solvent atoms to equilibrate water molecules keeping the solute restrained. Finally, a production run was performed to generate a 1microsecond trajectory with a total of 20.000 collected. Post HPC-run analysis of the results was performed.

In a second step a High Performance Computing (HPC) simulation was conducted to virtual screen the Safe in Man (SIM) library, containing commercialized and under development drugs, already proved safe in man (> 10.000 drugs), against the 3C-like protease of SARS-CoV-2, on both the crystal structures and the most 100 relevant conformations extracted from MD runs.

The SIM library is a Dompé dataset containing about 10.000 pharmaceutical compounds, built merging the list of drugs launched or under active development in several clinical phases with molecules in early phases (biological testing and preclinical) matching the query "CoV Inhibitors" as mechanism of action. Both lists derived from an Integrity database search (https://clarivate.com/cortellis/solutions/pre-clinical-intelligence-analytics/). All the compounds have been classified based on the mechanism of action of the class (antibacterial, antiviral, antiparasitic, antifungal, etc...) and on merging information reported in the Integrity database with those included in the drug repositioning database "RepoDB". Also drug information provided by DrugCentral, related to pharmaceutical products, drug mode of action, indications and pharmacologic action, was integrated in Dompé drug virtual library, as well as information on drugs and drug targets reported in the DrugBank database.

The simulation was performed using LiGen™ (Ligand Generator), the de novo structure based virtual screening software, designed and developed to run on HPC architectures, which represent the most relevant tool of the EXSCALATE platform. LiGenTM is formed by a set of tools that can be combined in a user-defined manner to generate project centric workflows. In particular, LiGenDock is a docking module using LiGenScore to compute the scoring function and the LiGenPass and LiGenPocket modules to obtain the 3D structure of the binding site. Both the docking algorithm implemented in LiGen, the pharmacophoric docking (LiGenPh4) and the geometrical docking (LiGenGeodock), as well as the different scoring functions calculated, were used in this study to explore different protocols of Virtual Screening (VS) and select the best one in terms of performance.

Usually, the performance of a VS protocol is assessed by evaluating its capacity to recognize the active molecules among a large number of inactive decoys, where usually the active molecules represent the 1% of the total number of compounds. The performances of the tested VS strategy was assessed by evaluating the capacity to correctly rank molecules which are endowed with antiviral activity, and in particular with a known effect against coronaviruses. Following this approach, about 130 molecules were labelled as active compounds.

The VS protocol performance was thus evaluated comparing the screening results of the SIM library on the crystal structure of 3C-like protease of SARS-CoV-2 and on the most 100 relevant conformations extracted from MD runs, by using different docking algorithms and different scoring function. The conditions showing the best capacity to recognize the active molecules were used to prioritize the total list of screened molecules and select the top scored compounds, according to the score value (Csopt), that predicts the binding affinity of the molecules in the protein binding site. In particular, the top 1.5% of the total number of screened molecules was selected as corresponding to a set of 160 molecules with a Csopt value higher than 6.3. The compounds list is reported in the table.

The list of selected compounds, with their docking score and their class with regard to their known mechanism of action, is reported in Table 2 below.

**Table 2**

| **ID** | **Chemical Name** | **Docking score** | **Class** |
|---|---|---|---|
| 1 | N1-[4-[5-(2-Phenanthryl)-3-(trifluoromethyl)-1H-pyrazol-1-yl]phenyl]glycinamide | 6.96 | Antiviral |
| 2 | N-(5-[3-[7-(3-Fluorophenyl)-1H-imidazo[4,5-c]pyridin-2-yl]-1 H-indazol-5-yl]pyridin-3-yl)-3-methylbutanamide | 6.96 | other (no antii nfective) |
| 3 | N-[3-Chloro-4-[(12aS)-3,4,6,11,12,12a-hexahydro-1H-[1,4]oxazino[3,4-c][1,4]benzodiazepin-11-ylcarbonyl]phenyl]biphenyl-2-carboxamide hydrochloride | 6.90 | other (no antii nfective) |
| 4 | 2-(5-[(Z)-[1-(4-tert-Butylphenyl)-5-oxo-3-phenyl-1,5-dihydro-4H-pyrazol-4-ylidene]methyl]furan-2-yl)-4-chlorobenzoic acid | 6.85 | coronavirus (3C-Like Protease) |
| 5 | 2-[1-(4-tert-Butylbenzyl)-5-(3-methylphenyl)-1 H-indol-3-yl]-2-oxoacetic acid | 6.81 | other (no antiinfective) |
| 6 | N'-[4-[4-[2-[3-(Dimethylaminomethyl)phenyl]-1H-pyrrolo[2,3-b]pyridin-4-yl]-1-ethyl-1 H-pyrazol-3-yl]phenyl]-N, N-di methylurea | 6.75 | other (no antii nfective) |
| 7 | (4S,5R)-5-[3,5-Bis(trifluoromethyl)phenyl]-3-([2-[4-fluoro-2-methoxy-5-(propan-2-yl)phenyl]-5,5-dimethylcyclohex-1-en-1-yl]methyl)-4-methyl-1,3-oxazolidin-2-one | 6.75 | other (no antii nfective) |
| 8 | [2-[6-(2-Ethyl-5-fluoro-4-hydroxyphenyl)-1H-indazol-3-yl]-1,4,6,7 -tetrahydro-5H-imidazo[4,5-c]pyridin-5-yl][5-(piperidin-1-yl)pyrazin-2-yl]methanone | 6.72 | other (no antii nfective) |
| 9 | 2-Bromoergocryptine monomethanesulfonate | 6.71 | other (no antii nfective) |
| 10 | (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester; (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydronicotinic acid ethyl ester | 6.71 | Antiviral |
| 11 | 4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester | 6.71 | other (no antii nfective) |
| 12 | 4-Methyl-N-[3-(4-methyl-1H-imidazol-1-yl)-5-(trifluoromethyl)phenyl]-3-[4-(3-pyridyl)pyrimidin-2-ylamino]benzamide hydrochloride monohydrate | 6.70 | other (no antii nfective) |
| 13 | 4-Chloro-2-[5-[[1-(4-fluorophenyl)-5-oxo-3-phenyl-1,5-dihydro-4H-pyrazol-4-ylidene]methyl]-2-furyl]benzoic acid | 6.70 | coronavirus (3C-Like Protease) |
| 14 | 2-Chloro-6-methyl-N'-[[4-methyl-3-(quinolin-3-ylethynyl)phenyl]carbonyl]benzohydrazide | 6.70 | other (no antii nfective) |
| 15 | (±)-N-(4-Amino-3,4-dioxo-1-phenylbutan-2-yl)-2-[3-(4-fluorophenyl)-1H-pyrazol-1-yl]pyridine-3-carboxamide | 6.69 | other (no antii nfective) |
| 16 | 3(R)-(2,3-Dihydro-1-benzofuran-5-yl)-2-[5-(2-pyridyl)pyrimidin-2-yl]-2,3,4,9-tetrahydro-1 H-pyrrolo[3,4-b]quinolin-9-one | 6.68 | other (no antii nfective) |
| 17 | 4-Chloro-2-[5-[(5-oxo-1,3-diphenyl-1,5-dihydro-4H-pyrazol-4-ylidene)methyl]-2-furyl]benzoic acid | 6.68 | coronavirus (3C-Like Protease) |
| 18 | 4-Amino-N-[1-(3-chloro-2-fluoroanilino)-6-methylisoquinolin-5-yl]thieno[3,2-d]pyrimidine-7-carboxamide | 6.66 | other (no antii nfective) |
| 19 | (S,S)-6,6'-[Carbonylbis(5-imino-1 H-indole-2-carbonyl)]bis[8-(chloromethyl)-1-methyl-3,6,7,8-tetrahydrobenzo[1,2-b:4,3-b']dipyrrol-4-ol]; (S,S)-1,3-Bis[2-[1-(Chloromethyl)-5-hydroxy-8-methyl-1,2,3,6-tetrahydrobenzo[1,2-b:4,3-b']dipyrrol-3-ylcarbonyl]-1 H-indol-5-yl]urea | 6.65 | other (no antii nfective) |
| 20 | N-[3-[6-[4-[1,4-Dimethyl-3-oxopiperazin-2(R)-yl]phenylamino]-4-methyl-5-oxo-4,5-dihydropyrazin-2-yl]-2-methylphenyl]-4,5,6,7-tetrahydro-1-benzothiophene-2-carboxamide | 6.65 | other (no antii nfective) |
| 21 | 2-Hydroxy-5-[4-[3-[4-(2-methylimidazo[4,5-c]pyridin-1-ylmethyl)piperidin-1-yl]-3-oxo-1-phenyl-1 (Z)-propenyl]phenylazo]benzoic acid | 6.65 | other (no antii nfective) |
| 22 | (-)-2-[3-(1,4-Dimethyl-1H-1,2,3-triazol-5-yl)-5-[(S)-phenyl(tetrahydro-2H-pyran-4-yl)methyl]-5H-pyrido[3,2-b]indol-7-yl]propan-2-ol | 6.64 | other (no antii nfective) |
| 23 | 4-[(2R,4R)-4-[[1-(2,2-Difluoro-2H-1,3-benzodioxol-5-yl)cyclopropane-1-carbonyl]amino]-7-(difluoromethoxy)-3,4-dihydro-2H-1-benzopyran-2-yl]benzoic acid | 6.63 | other (no antii nfective) |
| 24 | 2-[5-[(5-Oxo-1,3-diphenyl-1,5-dihydro-4H-pyrazol-4-ylidene)methyl]furan-2-yl]benzoic acid | 6.63 | coronavirus (3C-Like Protease) |
| 25 | 1-[2-[1-[3,5-Bis(trifluoromethyl)benzyl]-5-(4-pyridyl)-1 H-1,2,3-triazol-4-yl]pyridin-3-yl]-1-(2-chlorophenyl)methanone | 6.63 | other (no antii nfective) |
| 26 | 6-[(5S,9R)-9-(4-Cyanophenyl)-3-(3,5-dichlorophenyl)-1-methyl-2,4-dioxo-1,3,7-triazaspiro[4.4]non-7-yl]pyridine-3-carboxylic acid | 6.63 | other (no antii nfective) |
| 27 | (6S)-6-(2-Hydroxy-2-methylpropyl)-3-[(1S)-1-[4-(1-methyl-2-oxo-1,2-dihydropyridin-4-yl)phenyl]ethyl]-6-phenyltetrahydro-2H-1,3-oxazin-2-one hydrate | 6.63 | other (no antii nfective) |
| 28 | Carbonic acid cholest-5-en-3beta-yl 3,5-dichloro-2-methoxy-6-(trichloromethyl)pyridin-4-yl diester | 6.62 | other (no antii nfective) |
| 29 | 3-[3-[1-(N-Isopropyl-N-phenylcarbamoylmethyl)-2,4-dioxo-5-phenyl-2,3,4,5-tetrahydro-1H-1,5-benzodiazepin-3(S)-yl]ureido]benzoic acid | 6.62 | other (no antii nfective) |
| 30 | 2-[3-[N-Methyl-N-[1-(naphthalen-2-ylcarbonyl)piperidin-4-yl]carbamoyl]naphthalen-2-yl]-1-(1-naphthyl)-2-oxoethylphosphonic acid | 6.61 | other (no antiinfective) |
| 31 | 2-Propyl-1-[2'-(1 H-tetrazol-5-yl)biphenyl-4-ylmethyl]-4-[2-(trifluoroacetyl)pyrrol-1-yl]imidazole-5-carboxylic acid | 6.61 | other (no antiinfective) |
| 32 | 4'-[4-Methyl-6-(1-methyl-1 H-benzimidazol-2-yl)-2-propyl-1 H-benzimidazol-1-ylmethyl]biphenyl-2-carboxylic acid | 6.60 | other (no antiinfective) |
| 33 | 5-Chloro-N-(3-cyclopropyl-5-[(cis-3,5-dimethylpiperazin-1-yl)methyl]phenyl)-4-(6-methyl-1 H-indol-3-yl)pyrimidin-2-amine hydrate | 6.60 | other (no antiinfective) |
| 34 | 11-Keto-beta-boswellic acid; 3alpha-Hydroxyurs-12-en-11-keto-23-oic acid | 6.60 | other (no antiinfective) |
| 35 | N-(4-tert-Butylphenyl)-N-[2-[(2-methylpropyl)amino]-2-oxo-1-(pyridin-3-yl)ethyl]-1H-imidazole-4-carboxamide | 6.60 | coronavirus (3C-Like Protease) |
| 36 | 5-[N-[5-[N-(Cycloheptylmethyl)carbamoyl]-1 H-benzi midazol-6-ylcarbonyl]-2-fluoro-L-phenylalanylamino]isophthalic acid; 5-[N-[5-[N-(Cycloheptylmethyl)carbamoyl]-1 H-benzimidazol-6-ylcarbonyl]-2-fluoro-L-phenylalanylamino]benzene-1,3-dicarboxylic acid | 6.59 | other (no antiinfective) |
| 37 | 21-[4-[2,6-Bis(pyrrolidino)pyrimidin-4-yl]piperazin-1-yl]-16alpha-methyl-pregna-1,4,9(11)-triene-3,20-dione methanesulfonate hydrate; 16alphaMethyl-21-[4-[2,6-bis(pyrrolidino)-4-pyrimidinyl]-1-piperazinyl]pregna-1,4,9(11)triene-3,20-dione monomethanesulfonate hydrate | 6.59 | other (no antiinfective) |
| 38 | 3-[5-Oxo-3-phenyl-4-[(5-phenylfuran-2-yl)methylidene]-4,5-dihydro-1 H-pyrazol-1-yl]benzoic acid | 6.59 | coronavirus (3C-Like Protease) |
| 39 | 2-[2-[2-(2-Chlorophenyl)propan-2-yl]-1-[3'-(methylsulfonyl)biphenyl-4-yl]-1 H-imidazol-4-yl]propan-2-ol | 6.59 | other (no antiinfective) |
| 40 | 4-[4-[3-[3-tert-Butyl-1-(6-quinolinyl)-1H-pyrazol-5-yl]ureido]-3-fluorophenoxy]-N-methylpyridine-2-carboxamide tosylate salt | 6.59 | other (no antiinfective) |
| 41 | Benzoic acid 4-chloro-3-[5-methyl-3-[4-[2-(1-pyrrolidinyl)ethoxy]phenylamino]-1,2,4-benzotriazin-7-yl]phenyl ester | 6.59 | other (no antiinfective) |
| 42 | N-[3-Fluoro-4-[[11 C]methyloxy]phenyl]-6-[(2R)-2-(3-fluorophenyl)pyrrolidin-1-yl]imidazo[1,2-b]pyridazine-3-carboxamide | 6.58 | other (no antiinfective) |
| 43 | 4-Fluoro-2-(4-fluorophenyl)-N-methyl-5-(2-methyl-5-[[1-(pyrimidin-2-yl)cyclopropyl]carbamoyl]phenyl)-1-benzofuran-3-carboxamide | 6.58 | Antiviral |
| 44 | 6-Methyl-N5-[3-(1 H-purin-6-yl)pyridin-2-yl]-N1-[3-(trifluoromethoxy)phenyl]isoquinoline-1,5-diamine | 6.58 | other (no antiinfective) |
| 45 | (aR)-6-Fluoro-7-(2-fluoro-6-hydroxyphenyl)-1-[4-methyl-2-(propan-2-yl)pyridin-3-yl]-4-[(2S)-2-methyl-4-(prop-2-enoyl)piperazin-1-yl]pyrido[2,3-d]pyrimidin-2(1 H)-one | 6.58 | other (no antiinfective) |
| 46 | N-(3-Fluoro-4-[[7-(2-hydroxy-2-methylpropoxy)quinolin-4-yl]oxy]phenyl)-1,5-dimethyl-3-oxo-2-phenyl-2,3-dihydro-1 H-pyrazole-4-carboxamide 4-methylbenzenesulfonate | 6.58 | other (no antiinfective) |
| 47 | 4-[2-[4-(4-Ethylphenyl)-2,2-dimethyl-2H-1-benzothiopyran-6-yl]ethynyl]benzoic acid | 6.58 | other (no antiinfective) |
| 48 | (2E)-3-(6-Aminopyridin-3-yl)-N-([5-[4-(4,4-difluoropiperidine-1-carbonyl)phenyl]-7-(4-fluorophenyl)-1-benzofuran-2-yl]methyl)prop-2-enamide | 6.57 | other (no antiinfective) |
| 49 | 3-Chloro-N-[1-(N,N-dimethylglycinamido)-3-[4-[8-[1(S)-hydroxyethyl]imidazo[1,2-a]pyridin-2-yl]phenyl]propan-2(S)-yl]-4-isopropoxybenzamide | 6.56 | other (no antiinfective) |
| 50 | Phosphoric acid (6aR,9aS)-3-anilino-2-[[4-(6-fluoropyridin-2-yl)phenyl]methyl]-5-methyl-5,6a,7,8,9,9a-hexahydrocyclopenta[4,5]imidazo[1,2-a]pyrazolo[4,3-e]pyrimidin-4(2H)-one | 6.56 | other (no antiinfective) |
| 51 | (4R)-1-[[3-Acetyl-5-(2-methylpyrimidin-5-yl)-1 H-indazol-1-yl]acetyl]-N-(6-bromopyridin-2-yl)-4-fluoro-L-prolinamide | 6.55 | other (no antiinfective) |
| 52 | 1-[3-Bromo-2-[2-(trifluoromethylsulfonamido)phenyl]benzofuran-5-ylmethyl]-4-cyclopropyl-2-ethyl-1 H-imidazole-5-carboxamide | 6.54 | other (no antiinfective) |
| 53 | 2-(4-Fluorophenyl)-5-(11-fluoro-6H-pyrido[2',3':5,6][1,3]oxazino[3,4-a]indol-2-yl)-6-[(methanesulfonyl)(methyl)amino]-N-methyl-1-benzofuran-3-carboxamide | 6.54 | Antiviral |
| 54 | Bis(4,4'-dimethyl-2,2'-bipyridine) 2-[[1(2),2(2):2(5),3(2)-terthiophen]-1(5)-yl]-1 H-imidazo[4,5-f][1,10]phenanthroline dichloridoruthenium | 6.54 | other (no antiinfective) |
| 55 | N-[4-[2-[4-[3-Ethoxy-1 (E)-propenyl]phenyl]-4-[4-(isopropylamino)phenyl]-1 H-imidazol-5-yl]phenyl]-N-isopropylamine; 2-[4-[3-Ethoxy-1(E)-propenyl]phenyl]-4,5-bis[4-(isopropylamino)phenyl]-1 H-imidazole | 6.54 | other (no antiinfective) |
| 56 | Phosphoric acid [(3beta,5alpha,24R)-stigmastan-3-yl] L-ascorbic acid-2-O-yl diester disodium salt; 2-O-[Hydroxy[(3beta,5alpha,24R)-stigmastan-3-O-yl]phosphoryl]-L-ascorbic acid disodium salt | 6.53 | other (no antiinfective) |
| 57 | N-[4-Methyl-3-[4-(3-pyridyl)pyrimidin-2-ylamino)phenyl]-3,5-bis(trifluoromethyl)benzamide | 6.53 | other (no antiinfective) |
| 58 | 1-(6,7-Dihydro-5H-benzo[6,7]cyclohepta[1,2-c]pyridazin-3-yl)-N3-[7(S)-(1-pyrrolidinyl)-6,7,8,9-tetrahydro-5H-benzocycloheptene-2-yl]-1H-1,2,4-triazole-3,5-diamine | 6.53 | other (no antiinfective) |
| 59 | 5-[5-Phenyl-4-(pyridin-2-ylmethylamino)quinazolin-2-yl]pyridine-3-sulfonamide | 6.53 | other (no antiinfective) |
| 60 | N-[2-(2,1,3-Benzothiadiazol-5-ylamino)-6-(2,6-dichlorophenyl)pyrido[2,3-d]pyrimidin-7-yl]-N'-tert-butyl urea | 6.53 | other (no antiinfective) |
| 61 | N-[4-(2-Methyl-1,4,5,6-tetrahydroimidazo[4,5-d][1 ]benzazepin-6-ylcarbonyl)phenyl]biphenyl-2-carboxamide hydrochloride | 6.53 | other (no antiinfective) |
| 62 | N-[4-[3-(N-Benzyl-N-methylaminomethyl)-7-(2,6-difluorobenzyl)-5-isobutyryl-4-oxo-4,7-dihydrothieno[2,3-b]pyridin-2-yl]phenyl]-1-hydroxycyclopropanecarboxamide | 6.52 | other (no antiinfective) |
| 63 | 2-(3-Chloro-4-fluorobenzyl)-8-ethyl-10-hydroxy-6(S)-methyl-4-(5-methyl-1,1-dioxido-1,2,5-thiadiazolidin-2-yl)-1,2,6,7,8,9-hexahydropyrazino[1',2':1,5]pyrrolo[2,3-d]pyridazine-1,9-dione | 6.52 | Antiviral |
| 64 | (7bR,8aS)-N-[2-[(1,2,4,5,8,8a-Hexahydro-7-methyl-4-oxocyclopropa[c]pyrrolo[3,2-e]indol-2-yl)carbonyl]-1H-indol-5-yl]-2-benzofurancarboxamide | 6.52 | other (no antiinfective) |
| 65 | N-(2,4-Di-tert-butyl-5-hydroxyphenyl)-4-oxo-1,4-dihydroquinoline-3-carboxamide | 6.52 | other (no antiinfective) |
| 66 | N-(4-tert-Butylphenyl)-N-[2-(cyclohexylamino)-2-oxo-1-(pyridin-3-yl)ethyl]-1 H-imidazole-4-carboxamide | 6.52 | coronavirus (3C-Like Protease) |
| 67 | 1-(3,4-Dimethylphenyl)-3-methyl-4,5-dihydro-1 H-pyrazole-4,5-dione 4-[4-[2-hydroxy-3'-(1 H-tetrazol-5-yl)biphenyl-3-yl]hydrazone choline salt | 6.51 | other (no antiinfective) |
| 68 | Piperazine-1,4-diylbis[[6-(1H-benzimidazol-2-yl)pyridin-2-yl]methanone] | 6.51 | other (no antiinfective) |
| 69 | 4,5,6,7-Tetrachloro-2',4',5',7'-tetraiodo-3-oxo-3H-spiro[2-benzofuran-1,9'-xanthene]-3',6'-diolate disodium salt | 6.51 | other (no antiinfective) |
| 70 | 3-(4-Chlorophenylamino)-10-(4-chlorophenyl)-2,10-dihydro-2-(isopropylimino)phenazine | 6.51 | Antibacterial |
| 71 | (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide | 6.50 | other (no antiinfective) |
| 72 | 4-[[(3S,4R,5S)-3-(3-Chloro-2-fluorophenyl)-4-(4-chloro-2-fluorophenyl)-4-cyano-5-(2,2-dimethylpropyl)-D-prolyl]amino]-3-methoxybenzoic acid | 6.50 | other (no antiinfective) |
| 73 | 1-(2-Naphthylmethyl)-3-[(4-nitrophenyl)imino]-2-oxoindoline-5-carboxamide | 6.50 | coronavirus (3C-Like Protease) |
| 74 | N-[3-(7-Methyl-1H-indazol-5-yl)-1-[4-(1-methylpiperidin-4-yl)piperazin-1-yl]-1-oxopropan-2(R)-yl]-4-(2-oxo-1,2-dihydroquinolin-3-yl)piperidine-1-carboxamide | 6.50 | other (no antiinfective) |
| 75 | 2-[2-Fluoro-4-[7-(2-methylpyridin-4-yl)imidazo[1,2-a]pyridin-3-yl]phenyl]-N-[3-(triffuoromethyl)phenyl]acetamide | 6.50 | other (no antiinfective) |
| 76 | N-(2-Fluoro-6-methylphenyl)-6-(4-[[5-methyl-2-(7-oxa-2-azaspiro[3.5]nonan-2-yl)pyridine-3-carbonyl]amino]benzoyl)-5,6-dihydro-4H-thieno[3,2-d][1]benzazepine-2-carboxamide | 6.50 | Antiviral |
| 77 | 2-[2-[2-[2(E)-[4-(Trifluoromethyl)phenyl]vinyl]-1H-benzimidazol-5-yl]phenyl]propan-2-ol | 6.49 | other (no antiinfective) |
| 78 | 4-Methyl-N-[3-(4-methyl-1H-imidazol-1-yl)-5-(trifluoromethyl)phenyl]-3-[4-(2-pyrazinyl)pyrimidin-2-ylamino]benzamide | 6.49 | other (no antiinfective) |
| 79 | 7-(4,7-Diazaspiro[2.5]octan-7-yl)-2-(2,8-dimethylimidazo[1,2-b]pyridazin-6-yl)-4H-pyrido[1,2-a]pyrimidin-4-one | 6.49 | other (no antiinfective) |
| 80 | 3-[(4-Methylphenyl)imino]-1-(2-naphthylmethyl)-2-oxoindoline-5-carboxamide | 6.49 | coronavirus (3C-Like Protease) |
| 81 | 4-(4-Methylpiperazin-1-ylmethyl)-N-[6-methyl-5-[4-(3-pyridyl)pyrimidin-2-ylamino]pyridin-3-yl]-3-(trifluoromethyl)benzamide methanesulfonate | 6.49 | other (no antiinfective) |
| 82 | 2-(5-Amino-6-oxo-2-phenyl-1,6-dihydropyrimidin-1-yl)-N-[1-(5-tert-butyl-1,3,4-oxadiazol-2-ycarbonyl)-2-methylpropyl]acetamide | 6.49 | other (no antiinfective) |
| 83 | N-[3-[5-(2-Aminopyrimidin-4-yl)-2-tert-butylthiazol-4-yl]-2-fluorophenyl]-2,6-difluorobenzenesulfonamide methanesulfonate | 6.48 | other (no antiinfective) |
| 84 | (OC-6-23)-[[3-(Dimethylamino)propyl]dimethylsilinolato-kappaO]hydroxy[29H,31H-phthalocyanato(2-)-kappaN29,kappaN30,kappaN31,kappaN32]silico n | 6.48 | other (no antiinfective) |
| 85 | Methanesulfonic acid 2-[3-[4-[(1 H-indazol-5-yl)amino]quinazolin-2-yl]phenoxy]-N-(propan-2-yl)acetamide | 6.48 | other (no antiinfective) |
| 86 | Sodium (2E)-3-[2-[1-[[[2-(5-bromopyrimidin-2-yl)-3-cyclopentyl-1-methyl-1H-indol-6-yl]carbonyl]amino]cyclobutyl]-1-methyl-1H-benzimidazol-6-yl]acrylate | 6.48 | Antiviral |
| 87 | [4(R),6(S),(E)]-6-[2-[4-(4-Fluorophenyl)-2-isopropyl-6-phenyl-3-pyridyl]vinyl]-4-hydroxytetrahydropyran-2-one | 6.48 | other (no antiinfective) |
| 88 | [1-(4-[[4-(4-Hydroxypiperidin-1-yl)phenyl]amino]-5-oxo-5,6-dihydropyrimido[4,5-d]pyridazin-2-yl)piperidin-4-yl]acetonitrile | 6.48 | other (no antiinfective) |
| 89 | N-[2-(Benzylamino)-2-oxo-1-(pyridin-3-yl)ethyl]-N-(4-tert-butylphenyl)-1H-imidazole-4-carboxamide | 6.47 | coronavirus (3C-Like Protease) |
| 90 | N6-(4,4-Dimethyl-4,5-dihydroisoxazol-2-yl)-N4-[3-methyl-4-([1,2,4]triazolo[1,5-a]pyridin-7-yloxy)phenyl]quinazoline-4,6-diamine | 6.47 | other (no antiinfective) |
| 91 | 5-Chloro-N2-[5-methyl-4-(piperidin-4-yl)-2-(propan-2-yloxy)phenyl]-N4-[2-(propan-2-ylsulfonyl)phenyl]pyrimidine-2,4-diamine | 6.47 | other (no antiinfective) |
| 92 | (2S)-tert-Butoxy[4-(2,3-dihydropyrano[4,3,2-de]quinolin-7-yl)-2-methylquinolin-3-yl]ethanoic acid | 6.47 | Antiviral |
| 93 | cis-3-[8-Amino-1-(2-phenylquinolin-7-yl)imidazo[1,5-a]pyrazin-3-yl]-1-methylcyclobutanol | 6.47 | other (no antiinfective) |
| 94 | (Z)-2-[4-[2-[1-[4-[(Propan-2-yl)oxy]phenyl]-1H-benzimidazol-5-yl]ethenyl]phenyl]propan-2-ol | 6.47 | Antiviral |
| 95 | 6alpha,9alpha-Difluoro-16alpha,17alpha-[4-(dimethylamino)phenylmethylenedioxy]-11beta-hydroxy-21-(3-benzamido-2-methylpropionyloxy)preqna-1,4-dien-3-one | 6.47 | other (no antiinfective) |
| 96 | N-Cyclopropyl-4-[7-[[(cis-3-hydroxy-3-methylcyclobutyl)methyl]amino]-5-(pyridin-3-yloxy)pyrazolo[1,5-a]pyrimidin-3-yl]-2-methylbenzamide hydrochloride | 6.47 | other (no antiinfective) |
| 97 | (+)-(R)-1-[4-(2-Chloro-5-fluorobenzamido)-3-methoxybenzoyl]-1,2,3,5-tetrahydrospiro[1- benzazepine-4,1'-[2]cyclopentene]-3'-carboxylic acid | 6.46 | other (no antiinfective) |
| 98 | 3-[4-[1-[3-Cyclopentyl-1-methyl-2-(2-pyridyl)-1H-indol-6-ylcarboxamido]cyclobutylcarboxamido]phenyl]-2-propenoic acid trifluoroacetate | 6.46 | Antiviral |
| 99 | 6-(1 H-Indazol-6-yl)-N-[4-(morpholin-4-yl)phenyl]imidazo[1,2-a]pyrazin-8-amine dimethanesulfonate | 6.46 | other (no antiinfective) |
| 100 | 2-[(1S)-1-[4-Amino-3-(3-fluoro-4-isopropoxyphenyl)-1H-pyrazolo[3,4-d]pyrimidin-1 - yl]ethyl]-6-fluoro-3-(3-fluorophenyl)-4H-chromen-4-one 4-methylbenzenesulfonate | 6.46 | other (no antiinfective) |
| 101 | 2-Butyl-5-[[11C]-methoxymethyl]-6-(1-oxidopyridin-2-yl)-3-[2'-(1 H-tetrazol-5-yl)biphenyl-4-ylmethyl]-3H-imidazo[4,5-b]pyridine | 6.46 | other (no antiinfective) |
| 102 | 3-Amino-3-methyl-N-[2-oxo-1-[2'-(1 H-tetrazol-5-yl)biphenyl-4-ylmethyl]-2,3,4,5-tetrahydro-1 H-1- benzazepin-3(R)-yl]butyramide | 6.46 | other (no antiinfective) |
| 103 | 4-([[(1S)-2-[(2E)-3-[3-Chloro-2-fluoro-6-(1 H-tetrazol-1-yl)phenyl]prop-2-enoyl]-5-(4-methyl-2-oxopiperazin-1-yl)-1,2,3,4-tetrahydroisoquinolin-1-yl]carbonyl]amino)benzoic acid hydrochloride | 6.45 | other (no antiinfective) |
| 104 | 5-[2-Hydroxy-3-[(2Z)-2-[3-methyl-5-oxo-1-(5,6,7,8-tetrahydronaphthalen-2-yl)-4,5-dihydro-1H-pyrazol-4-ylidene]hydrazino]phenyl]furan-2-carboxylic acid bis(2-aminoethanol) | 6.45 | other (no antiinfective) |
| 105 | 3-[4-(1-Cyclobutylpiperidin-4-yloxy)phenyl]-2-methyl-5-(trifluoromethyl)quinazolin-4(3H)-one | 6.45 | other (no antiinfective) |
| 106 | (R,S)-9-[Benzyloxy(hydroxy)phosphoryloxy]-14-methyl-5,14-dihydrobenz[5,6]isoindolo[2,1-b]isoquinoline-8,13-dione; Benzyl (±)-5,8,13,14-tetrahydro-14-methyl-8,13-dioxobenz[5,6]isoindolo[2,1-b]isoquinolin-9-yl hydrogen phosphate | 6.45 | other (no antiinfective) |
| 107 | 5-[6-[2,4-Bis(trifluoromethyl)phenyl]pyridazin-3-ylmethyl]-2-(2-fluorophenyl)-5H-imidazo[4,5-c]pyridine | 6.45 | Antiviral |
| 108 | 2,7-Bis[[(3R,5S)-3,5-dimethylpiperidin-1-yl]sulfonyl]-N,N'-dihydroxyanthracene-9,10-diimine | 6.45 | other (no antiinfective) |
| 109 | 4'-[2-Ethyl-4-methyl-6-(5,6,7,8-tetrahydroimidazo[1,2-a]pyridin-2-yl)benzimidazol-1-ylmethyl]biphenyl-2-carboxylic acid | 6.44 | other (no antiinfective) |
| 110 | (6S)-N-[(3S,5S,6R)-6-Methyl-2-oxo-1-(2,2,2-trifluoroethyl)-5-(2,3,6-trifluorophenyl)piperidin-3-yl]-2'-oxo-1',2',5,7-tetrahydrospiro[cyclopenta[b]pyridine-6,3'-pyrrolo[2,3-b]pyridine]-3-carboxamide | 6.44 | other (no antiinfective) |
| 111 | 5-(4-Cyclopropyl-1H-imidazol-1-yl)-2-fluoro-N-[6-(4-isopropyl-4H-1,2,4-triazol-3-yl)pyridin-2-yl]-4-methylbenzamide hydrochloride | 6.44 | other (no antiinfective) |
| 112 | N-(2-Bromo-3-oxospiro[3.5]non-1-en-1-yl)-4-(2,7-naphthyridin-1-ylamino)-L-phenylalanine ethyl ester | 6.44 | other (no antiinfective) |
| 113 | (6aR,9R,10aR)-N-[(2R,5S,10aS,10bS)-10b-Hydroxy-5-isobutyl-2-isopropyl-3,6-dioxoperhydrooxazolo[3,2-a]pyrrolo[1,2-d]pyrazin-2-yl]-7-methyl-4,6,6a,7,8,9,10,10a-octahydroindolo[4,3-fg]quinoline-9-carboxamide mesylate | 6.44 | other (no antiinfective) |
| 114 | 4-[9-Chloro-7-(2,6-difluorophenyl)-5H-pyrimido[5,4-d][2]benzazepin-2-ylamino]benzoic acid | 6.43 | other (no antiinfective) |
| 115 | 4-[4-[4-[4-Fluoro-3-(trifluoromethyl)phenyl]-1-methyl-1 H-imidazol-2-yl]piperidin-1-yl]-1H-pyrazolo[3,4-d]pyrimidine 4-methyl benzenesulfonate | 6.43 | other (no antiinfective) |
| 116 | Lup-20(29)-ene-3beta,28-diol | 6.43 | Antiviral |
| 117 | 1-(2-Naphthylmethyl)-2-oxo-3-(phenylimino)indoline-5-carboxamide | 6.43 | coronavirus (3C-Like Protease) |
| 118 | 3,3'-[(1,6-Dioxo-16-hexanediyl)diimino]bis[2,4,6-triiodobenzoic acid] diethyl ester | 6.43 | other (no antiinfective) |
| 119 | 2-(Piperidin-1-yl)-N-[4-(trifluoromethyl)phenyl]-7-[3-(trifluoromethyl)pyridin-2-yl]-6,7,8,9-tetrahydro-5H-pyrimido[4,5-d]azepin-4-amine sulfate | 6.43 | other (no antiinfective) |
| 120 | 4,5-Dichloro-N-[3-[1-(2,4-dichlorobenzyl)-5-fluoro-3-methyl-1 H-indol-7-yl]-2(E)-propenoyl]thiophene-2-sulfonamide | 6.43 | other (no antiinfective) |
| 121 | (2E)-N-(4-[[3-Chloro-4-(pyridin-2-ylmethoxy)phenyl]amino]-3-cyano-7-ethoxyquinolin-6-yl)-3-[(2R)-1-methylpyrrolidin-2-yl]prop-2-enamide (2Z)-but-2-enedioate | 6.43 | other (no antiinfective) |
| 122 | (2S)-N-[(4S,6S,7S)-7-[[(2,6-Dimethylphenoxy)acetyl]amino]-6-hydroxy-2-methyl-8-phenyloctan-4-yl]-3-methyl-2-(2-oxotetrahydropyrimidin-1(2H)-yl)butanamide | 6.43 | Antiviral |
| 123 | 4-[2,6-Di-tert-butyl-4-[1-(3,5-di-tert-butyl-4-hydroxyphenylsulfanyl)-1-methylethylsulfanyl]phenoxy]butyric acid | 6.42 | other (no antiinfective) |
| 124 | N-[2-(1H-1,2,4-Triazol-5-ylmethyl)-1,2,3,4-tetrahydroisoquinolin-6-yl]-4'-(trifluoromethyl)-1,1'-biphenyl-2-carboxamide | 6.42 | other (no antiinfective) |
| 125 | (1S)-1-(4-Fluorophenyl)-1-(2-[4-[6-(1-methyl-1H-pyrazol-4-yl)pyrrolo[2,1-f][1,2,4]triazin-4-yl]piperazin-1-yl]pyrimidin-5-yl)ethan-1-amine | 6.42 | other (no antiinfective) |
| 126 | 2-Hydroxy-N,N,N-trimethylethan-1-aminium (3beta,18alpha)-3-hydroxy-11-oxoolean-12-en-30-oate | 6.42 | other (no antiinfective) |
| 127 | N-[4-[3-(2-Aminopyrimidin-4-yl)pyridin-2-yloxy]phenyl]-4-(4-methylthien-2-yl)phthalazin-1-amine | 6.42 | other (no antiinfective) |
| 128 | 2-Hydroxy-1-[2-[[9-(trans-4-methylcyclohexyl)-9H-pyrido[4',3':4,5]pyrrolo[2,3-d]pyrimidin-2-yl]amino]-7,8-dihydro-1,6-naphthyridin-6(5H)-yl]ethanone | 6.42 | other (no antiinfective) |
| 129 | 9-Isopropyl-N2-[4-(4-methylpiperazin-1-yl)phenyl]-N8-(3-pyridyl)-9H-purine-2,8-diamine | 6.41 | other (no antiinfective) |
| 130 | 1(RS)-N-[4-[N-(4-Chlorophenylsulfonyl)carbamoyl]benzoyl]-L-valyl-N-[2-methyl-1-(trifluoroacetyl)propyl]-L-prolinamide | 6.41 | other (no antiinfective) |
| 131 | 2-Amino-5-cyano-6-methyl-4(R)-(3-phenylquinolin-5-yl)-1,4-dihydropyridine-3-carboxylic acid isopropyl ester | 6.41 | other (no antiinfective) |
| 132 | N-[3-tert-Butyl-1-(4-methylphenyl)-1H-pyrazol-5-yl]-N'-[4-[6-(morpholin-4-ylmethyl)pyridin-3-yl]naphthalen-1-yl]urea | 6.41 | other (no antiinfective) |
| 133 | 5-Fluoro-N-[2-[1-methylpyrrolidin-2(R)-yl]ethyl]-3-oxo-6-[3-(2-pyrazinyl)pyrrolidin-1-yl]-3H-benzo[b]pyrido[3,2,1-kl]phenoxazine-2-carboxamide | 6.41 | other (no antiinfective) |
| 134 | (2R,4aS,6aS,12bR,14aS,14bR)-10-Hydroxy-2,4a,6a,9, 12b, 14a-hexamethyl-11-oxo-1,2,3,4,4a,5,6,6a,11,12b,13,14,14a,14b-tetradecahydropicene-2-carboxylic acid L-threonine cocrystal | 6.40 | other (no antiinfective) |
| 135 | (3S,4aS,8aS)-N-tert-Butyl-2-[2(R)-hydroxy-3(R)-(3-hydroxy-2-methylbenzamido)-4-(phenylsulfanyl)butyl)perhydroisoquinoline-3-carboxamide methanesulfonate | 6.40 | coronavirus (HIV-1 Protease) |
| 136 | 9-Acetoxy-2-(1,2-dimethylpropyl)-8-(hexopyranosyloxy)-2,4a,7,7,10a,12a-hexamethyl-1,2,3,4,4a,5,6,6a,7,8,9,10,10a,11,12,12a-hexadecahydrochrysene-1-carboxylic acid | 6.40 | Antibacterial |
| 137 | (+)-2-Amino-N-[(1S)-1-[8-[(1-methyl-1H-pyrazol-4-yl)ethynyl]-1-oxo-2-phenyl-1,2-dihydroisoquinolin-3-yl]ethyl]pyrazolo[1,5-a]pyrimidine-3-carboxamide | 6.40 | other (no antiinfective) |
| 138 | 4"'-O-Methyldigoxin; (3beta,5beta,12beta,14beta)-[O-(2,6-Dideoxy-4-O-methyl-D-ribo-hexopyranosyl)-(1--4)-O-(2,6-dideoxy-beta-D-ribo-hexopyranosyl)-(1--4)-2,6-dideoxy-D-ribo-hexopyranosyloxy]-12,14-dihydroxy-card-20(22)-enolide | 6.40 | other (no antiinfective) |
| 139 | N-[2-Fluoro-5-[3-[2-(2-pyridyl)vinyl]-1H-indazol-6-ylamino]phenyl]-1,3-dimethyl-1 H-pyrazole-5-carboxamide | 6.40 | other (no antiinfective) |
| 140 | N-(4-tert-Butylphenyl)-N-[2-(cyclopentylamino)-2-oxo-1 -(pyridin-3-yl)ethyl]-1 H-imidazole-4-carboxamide | 6.40 | coronavirus (3C-Like Protease) |
| 141 | 7-[7-[(3bR,4aS)-3-Methyl-8-oxo-4,4a,5,8-tetrahydrocyclopropa[c]pyrrolo[3,2-e]indol-6(1H)-ylcarbonyl]-4-hydroxy-5-methoxy-1,2,3,6-tetrahydropyrrolo[3,2-e]indol-3-ylcarbonyl]-4-hydroxy-5-methoxy-1,2,3,6-tetrahydropyrrolo[3,2-e]indole-3-carboxamide | 6.39 | antibacterial |
| 142 | (6bS,8aS,11R,12aR,12bS,14aR)-3-Hydroxy-4,6b,8a,11,12b,14a-hexamethyl-2,6b,7,8,8a,9,10,11,12,12a,12b,13,14,14a-tetradecahydropicene-2,10-dione | 6.39 | coronavirus (3C-Like Protease) |
| 143 | (aR)-(2S)-6-Fluoro-5-[3-[(aS)-8-fluoro-1-methyl-2,4-dioxo-1,4-dihydroquinazolin-3(2H)-yl]-2-methylphenyl]-2-(2-hydroxypropan-2-yl)-2,3,4,9-tetrahydro-1 H-carbazole-8-carboxamide | 6.39 | other (no antiinfective) |
| 144 | 2-Benzyl-5-(4-chlorophenyl)-6-[4-(methylsulfanyl)phenyl]pyridazin-3(2H)-one | 6.38 | other (no antiinfective) |
| 145 | 3-[3-Carboxy-2-methyl-1-[(Z)-5,5,8,8-tetramethyl-5,6,7,8-tetrahydroanthracen-2-ylmethylene]-2(Z)-propenyl]benzoic acid; 4-(3-Carboxyphenyl)-3-methyl-5-(5,5,8,8-tetramethyl-5,6,7,8-tetrahydroanthracen-2-yl)-2(Z),4(Z)-pentadienoic acid | 6.38 | other (no antiinfective) |
| 146 | (S)-N-[1-(2-Fluorophenyl)-4-oxo-3,4,6,7-tetrahydropyrrolo[3,2,1-jk][1,4]benzodiazepin-3-yl]-1H-indole-2-carboxamide | 6.38 | other (no antiinfective) |
| 147 | 2',6-Difluoro-5'-[3-(1-hydroxy-1-methylethyl)imidazo[1,2-b][1,2,4]triazin-7-yl]biphenyl-2-carbonitrile | 6.38 | other (no antiinfective) |
| 148 | 4-(Dimethylamino)-N-[3-[4-[2-hydroxy-1(S)-phenylethylamino]-6-phenylfuro[2,3-d]pyrimidin-5-yl]phenyl]-2-butenamide | 6.38 | other (no antiinfective) |
| 149 | N'-[4-[4-(Dimethylamino)piperidin-1-ylcarbonyl]phenyl]-N-[4-[4,6-di(4-morpholinyl)-1,3,5-triazin-2-yl]phenyl]urea | 6.38 | other (no antiinfective) |
| 150 | 6-(2-Chlorophenyl)-9-[(4-methoxyphenyl)carbamoyl]-1-methyl-4,7,8,10-tetrahydropyrido[4',3':4,5]thieno[3,2-f][1,2,4]triazolo[4,3-a][1,4]diazepine | 6.37 | other (no antiinfective) |
| 151 | N-(1',2-Dihydroxy-1,2'-binaphthalen-4'-yl)-4-methoxybenzenesulfonamide | 6.37 | other (no antiinfective) |
| 152 | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid 2-aminoethanol (1:2) | 6.36 | other (no antiinfective) |
| 153 | (11beta,16beta)-9-Fluoro-1',4'-dihydro-11,21-dihydroxy-2'H-naphtho[2',3':16,17]pregna-1,4-diene-3,20-dione monohydrate; 9-Fluoro-1',4'-dihydro-11beta,21-dihydroxy-2'betaH-naphtho[2',3':16,17]pregna-1,4-diene-3,20-dione monohydrate | 6.36 | other (no antiinfective) |
| 154 | N-(3-[5-[(1-Ethylpiperidin-4-yl)(methyl)amino]-3-(pyrimidin-5-yl)-1 H-pyrrolo[3,2-b]pyridin-1-yl]-2,4-difluorophenyl)propane-1-sulfonamide | 6.35 | other (no antiinfective) |
| 155 | N-Cycloheptyl-N-(9H-fluoren-2-ylmethyl)-N'-(2,4,6-trimethylphenyl)urea | 6.35 | other (no antiinfective) |
| 156 | 5-[(1 R,3R)-1-(1-Ethyl-1H-pyrazol-4-yl)-3-[4-(5-fluoropyridin-2-yl)-1 H-imidazol-2-yl]-2,3,4,9-tetrahydro-1 H-beta-carbolin-1-yl]-3-methyl-1,3,4-oxadiazol-2(3H)-one | 6.34 | other (no antiinfective) |
| 157 | N-(5-Ethyl-2,4-dimethylphenyl)-4-[3-fluoro-4-(4-methyl-1 H-imidazol-1-yl)phenyl]thiazol-2-amine | 6.33 | other (no antiinfective) |
| 158 | N-[2-(Diethylamino)ethyl]-2,4-dimethyl-5-[2-oxo-5-(2-phenylthiazol-4-yl)-2,3-dihydro-1 H-indol-3-ylidenemethyl]-1 H-pyrrole-3-carboxamide | 6.33 | other (no antiinfective) |
| 159 | N-(4-Oxo-3,4-dihydroquinazolin-2-yl)-L-prolyl-N-methyl-L-3-(2-naphthyl)alanine N-benzyl-N-methylamide | 6.31 | other (no antiinfective) |
| 160 | (5S,6R,8R)-3'-(Methylpolyehtyleneglycol)-5-methyl-7,8,14,15-tetrahydro-2'H,4'H,13H-spiro[5,8-epoxy-4b,8a,14-triazadibenzo[b,h]cycloocta[1,2,3,4-jkl]cyclopenta[e]-as-indacene-6,5'-[1,3]oxazolidine]-2',4',13-trione | 6.29 | other (no antiinfective) |

## Claims

1. A compound inhibitor of the 3C-like protease of SARS-CoV-2, for use in the treatment of COVID-19, selected from compounds 1 to 160 below:
| **ID** | **Chemical Name** | **Generic Name (GN)** |
|---|---|---|
| 1 | N1-[4-[5-(2-Phenanthryl)-3-(trifluoromethyl)-1H-pyrazol-1-yl]phenyl]glycinamide | |
| 2 | N-(5-[3-[7-(3-Fluorophenyl)-1H-imidazo[4,5-c]pyridin-2-yl]-1 H-indazol-5-yl]pyridin-3-yl)-3-methylbutanamide | Adavivint (former INN); Lorecivivint (USAN; Rec INN) |
| 3 | N-[3-Chloro-4-[(12aS)-3,4,6,11,12,12a-hexahydro-1 H-[1,4]oxazino[3,4-c][1,4]benzodiazepin-11-ylcarbonyl]phenyl]biphenyl-2-carboxamide hyd roch loride | |
| 4 | 2-(5-[(Z)-[1-(4-tert-Butylphenyl)-5-oxo-3-phenyl-1,5-dihydro-4H-pyrazol-4-ylidene]methyl]furan-2-yl)-4-chlorobenzoic acid | |
| 5 | 2-[1-(4-tert-Butylbenzyl)-5-(3-methylphenyl)-1 H-indol-3-yl]-2-oxoacetic acid | Aleplasinin (Prop INN; USAN) |
| 6 | N'-[4-[4-[2-[3-(Dimethylaminomethyl)phenyl]-1H-pyrrolo[2,3-b]pyridin-4-yl]-1-ethyl-1 H-pyrazol-3-yl]phenyl]-N,N-dimethylurea | |
| 7 | (4S,5R)-5-[3,5-Bis(trifluoromethyl)phenyl]-3-([2-[4-fluoro-2-methoxy-5-(propan-2-yl)phenyl]-5,5-dimethylcyclohex-1-en-1-yl]methyl)-4-methyl-1,3-oxazolidin-2-one | Rocacetrapib (Rec INN) |
| 8 | [2-[6-(2-Ethyl-5-fluoro-4-hydroxyphenyl)-1 H-indazol-3-yl]-1 ,4,6,7-tetrahydro-5H-imidazo[4,5-c]pyridin-5-yl][5-(piperidin-1-yl)pyrazin-2-yl]methanone | |
| 9 | 2-Bromoergocryptine monomethanesulfonate | Bromocriptine mesilate (BANM; Rec INNM; USAN; JAN) |
| 10 | (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester; (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydronicotinic acid ethyl ester | Modipafant (BAN; Rec INN) |
| 11 | 4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester | |
| 12 | 4-Methyl-N-[3-(4-methyl-1H-imidazol-1-yl)-5-(trifluoromethyl)phenyl]-3-[4-(3-pyridyl)pyrimidin-2-ylamino]benzamide hydrochloride monohydrate | Nilotinib hydrochloride monohydrate (Rec INNM; USAN) |
| 13 | 4-Chloro-2-[5-[[1-(4-fluorophenyl)-5-oxo-3-phenyl-1,5-dihydro-4H-pyrazol-4-ylidene]methyl]-2-furyl]benzoic acid | |
| 14 | 2-Chloro-6-methyl-N'-[[4-methyl-3-(quinolin-3-ylethynyl)phenyl]carbonyl]benzohydrazide | |
| 15 | (±)-N-(4-Amino-3,4-dioxo-1-phenylbutan-2-yl)-2-[3-(4-fluorophenyl)-1 H-pyrazol-1-yl]pyridine-3-carboxamide | |
| 16 | 3(R)-(2,3-Dihydro-1-benzofuran-5-yl)-2-[5-(2-pyridyl)pyrimidin-2-yl]-2,3,4,9-tetrahydro-1H-pyrrolo[3,4-b]quinolin-9-one | |
| 17 | 4-Chloro-2-[5-[(5-oxo-1,3-diphenyl-1,5-dihydro-4H-pyrazol-4-ylidene)methyl]-2-furyl]benzoic acid | |
| 18 | 4-Amino-N-[1-(3-chloro-2-fluoroanilino)-6-methylisoquinolin-5-yl]thieno[3,2-d]pyrimidine-7-carboxamide | Belvarafenib (Rec INN) |
| 19 | (S,S)-6,6'-[Carbonylbis(5-imino-1 H-indole-2-carbonyl)]bis[8-(chloromethyl)-1-methyl-3,6,7,8-tetrahydrobenzo[1,2-b:4,3-b']dipyrrol-4-ol]; (S,S)-1,3-Bis[2-[1-(Chloromethyl)-5-hydroxy-8-methyl-1,2,3,6-tetrahydrobenzo[1,2-b:4,3-b']dipyrrol-3-ylcarbonyl]-1 H-indol-5-yl]urea | Bizelesin (USAN; Rec INN) |
| 20 | N-[3-[6-[4-[1,4-Dimethyl-3-oxopiperazin-2(R)-yl]phenylamino]-4-methyl-5-oxo-4,5-dihydropyrazin-2-yl]-2-methylphenyl]-4,5,6,7-tetrahydro-1-benzothiophene-2-carboxamide | |
| 21 | 2-Hydroxy-5-[4-[3-[4-(2-methylimidazo[4,5-c]pyridin-1-ylmethyl)piperidin-1-yl]-3-oxo-1-phenyl-1(Z)-propenyl]phenylazo]benzoic acid | Dersalazine (Rec INN) |
| 22 | (-)-2-[3-(1,4-Dimethyl-1 H-1,2,3-triazol-5-yl)-5-[(S)-phenyl(tetrahydro-2H-pyran-4-yl)methyl]-5H-pyrido[3,2-blindol-7-yl]propan-2-ol | |
| 23 | 4-[(2R,4R)-4-[[1-(2,2-Difluoro-2H-1,3-benzodioxol-5-yl)cyclopropane-1-carbonyl]amino]-7-(difluoromethoxy)-3,4-dihydro-2H-1-benzopyran-2-yl]benzoic acid | Galicaftor (USAN; Rec INN) |
| 24 | 2-[5-[(5-Oxo-1,3-diphenyl-1,5-dihydro-4H-pyrazol-4-ylidene)methyl]furan-2-yl]benzoic acid | |
| 25 | 1-[2-[1-[3,5-Bis(trifluoromethyl)benzyl]-5-(4-pyridyl)-1H-1,2,3-triazol-4-yl]pyridin-3-yl]-1-(2-chlorophenyl) methanone | Tradipitant (Prop INN; USAN) |
| 26 | 6-[(5S,9R)-9-(4-Cyanophenyl)-3-(3,5-dichlorophenyl)-1-methyl-2,4-dioxo-1,3,7-triazaspiro[4.4]non-7-yl]pyridine-3-carboxylic acid | |
| 27 | (6S)-6-(2-Hydroxy-2-methylpropyl)-3-[(1S)-1-[4-(1-methyl-2-oxo-1,2-dihydropyridin-4-yl)phenyl]ethyl]-6-phenyltetrahydro-2H-1,3-oxazin-2-one hydrate | |
| 28 | Carbonic acid cholest-5-en-3beta-yl 3,5-dichloro-2-methoxy-6-(trichloromethyl)pyridin-4-yl diester | 4-Demethyl-4-cholesteryloxycarbonylpenclomedine |
| 29 | 3-[3-[1-(N-Isopropyl-N-phenylcarbamoylmethyl)-2,4-dioxo-5-phenyl-2,3,4,5-tetrahydro-1H-1,5-benzodiazepin-3(S)-yl]ureido]benzoic acid | |
| 30 | 2-[3-[N-Methyl-N-[1-(naphthalen-2-ylcarbonyl)piperidin-4-yl]carbamoyl]naphthalen-2-yl]-1-(1-naphthyl)-2-oxoethylphosphonic acid | |
| 31 | 2-Propyl-1-[2'-(1 H-tetrazol-5-yl)biphenyl-4-ylmethyl]-4-[2-(trifluoroacetyl)pyrrol-1-yl]imidazole-5-carboxylic acid | |
| 32 | 4'-[4-Methyl-6-(1-methyl-1H-benzimidazol-2-yl)-2-propyl-1H-benzimidazol-1-ylmethyl]biphenyl-2-carboxylic acid | Telmisartan (USAN; Rec INN) |
| 33 | 5-Chloro-N-(3-cyclopropyl-5-[(cis-3,5-dimethylpiperazin-1-yl)methyl]phenyl)-4-(6-methyl-1 H-indol-3-yl)pyrimidin-2-amine hydrate | |
| 34 | 11-Keto-beta-boswellic acid; 3alpha-Hydroxyurs-12-en-11-keto-23-oic acid | 11-Keto-beta-boswellic acid |
| 35 | N-(4-tert-Butylphenyl)-N-[2-[(2-methylpropyl)amino]-2-oxo-1-(pyridin-3-yl)ethyl]-1 H-imidazole-4-carboxamide | |
| 36 | 5-[N-[5-[N-(Cycloheptylmethyl)carbamoyl]-1 H-benzimidazol-6-ylcarbonyl]-2-fluoro-L-phenylalanylamino]isophthalic acid; 5-[N-[5-[N-(Cycloheptylmethyl)carbamoyl]-1 H-benzimidazol-6-ylcarbonyl]-2-fluoro-L-phenylalanylamino]benzene-1,3-dicarboxylic acid | Gastrazole |
| 37 | 21-[4-[2,6-Bis(pyrrolidino)pyrimidin-4-yl]piperazin-1-yl]-16alpha-methyl-pregna-1,4,9(11)-triene-3,20-dione methanesulfonate hydrate; 16alpha-Methyl-21-[4-[2,6-bis(pyrrolidino)-4-pyrimidinyl]-1-piperazinyl]pregna-1,4,9(11 )triene-3,20-dione monomethanesulfonate hydrate | Tirilazad mesylate (Rec INNM; BAN; USAN) |
| 38 | 3-[5-Oxo-3-phenyl-4-[(5-phenylfuran-2-yl)methylidene]-4,5-dihydro-1 H-pyrazol-1-yl]benzoic acid | |
| 39 | 2-[2-[2-(2-Chlorophenyl)propan-2-yl]-1-[3'-(methylsulfonyl)biphenyl-4-yl]-1H-imidazol-4-yl]propan-2-ol | |
| 40 | 4-[4-[3-[3-tert-Butyl-1-(6-quinolinyl)-1H-pyrazol-5-yl]ureido]-3-fluorophenoxy]-N-methylpyridine-2-carboxamide tosylate salt | Rebastinib tosylate (USAN; Rec INN) |
| 41 | Benzoic acid 4-chloro-3-[5-methyl-3-[4-[2-(1-pyrrolidinyl)ethoxy]phenylamino]-1,2,4-benzotriazin-7-yl]phenyl ester | |
| 42 | N-[3-Fluoro-4-[[11 C]methyloxy]phenyl]-6-[(2R)-2-(3-fluorophenyl)pyrrolidin-1-yl]imidazo[1,2-b]pyridazine-3-carboxamide | |
| 43 | 4-Fluoro-2-(4-fluorophenyl)-N-methyl-5-(2-methyl-5-[[1-(pyrimidin-2-yl)cyclopropyl]carbamoyl]phenyl)-1-benzofuran-3-carboxamide | |
| 44 | 6-Methyl-N5-[3-(1 H-purin-6-yl)pyridin-2-yl]-N1-[3-(trifluoromethoxy)phenyl]isoquinoline-1,5-diamine | |
| 45 | (aR)-6-Fluoro-7-(2-fluoro-6-hydroxyphenyl)-1-[4-methyl-2-(propan-2-yl)pyridin-3-yl]-4-[(2S)-2-methyl-4-(prop-2-enoyl)piperazin-1-yl]pyrido[2,3-d]pyrimidin-2(1 H)-one | |
| 46 | N-(3-Fluoro-4-[[7-(2-hydroxy-2-methylpropoxy)quinolin-4-yl]oxy]phenyl)-1,5-dimethyl-3-oxo-2-phenyl-2,3-dihydro-1 H-pyrazole-4-carboxamide 4-methylbenzenesulfonate | Ningetinib Tosylate; Ningetinib (free base) |
| 47 | 4-[2-[4-(4-Ethylphenyl)-2,2-dimethyl-2H-1-benzothiopyran-6-yl]ethynyl]benzoic acid | |
| 48 | (2E)-3-(6-Aminopyridin-3-yl)-N-([5-[4-(4,4-difluoropiperidine-1-carbonyl)phenyl]-7-(4-fluorophenyl)-1-benzofuran-2-yl]methyl)prop-2-enamide | |
| 49 | 3-Chloro-N-[1-(N,N-dimethylglycinamido)-3-[4-[8-[1(S)-hydroxyethyl]imidazo[1,2-a]pyridin-2-yl]phenyl]propan-2(S)-yl]-4-isopropoxybenzamide | |
| 50 | Phosphoric acid (6aR,9aS)-3-anilino-2-[[4-(6-fluoropyridin-2-yl)phenyl]methyl]-5-methyl-5,6a,7,8,9,9a-hexahydrocyclopenta[4,5]imidazo[1,2-a]pyrazolo[4,3-e]pyrimidin-4(2H)-one | |
| 51 | (4R)-1-[[3-Acetyl-5-(2-methylpyrimidin-5-yl)-1H-indazol-1-yl]acetyl]-N-(6-bromopyridin-2-yl)-4-fluoro-L-prolinamide | Danicopan (USAN; Rec INN) |
| 52 | 1-[3-Bromo-2-[2-(trifluoromethylsulfonamido)phenyl]benzofuran-5-ylmethyl]-4-cyclopropyl-2-ethyl-1H-imidazole-5-carboxamide | Saprisartan (BAN; USAN; Rec INN) |
| 53 | 2-(4-Fluorophenyl)-5-(11-fluoro-6H-pyrido[2',3':5,6][1,3]oxazino[3,4-a]indol-2-yl)-6-[(methanesulfonyl)(methyl)amino]-N-methyl-1-benzofu ran-3-carboxamide | |
| 54 | Bis(4,4'-dimethyl-2,2'-bipyridine) 2-[[1(2),2(2):2(5),3(2)-terthiophen]-1(5)-yl]-1 H-imidazo[4,5-f][1,10]phenanthroline dichloridoruthenium | |
| 55 | N-[4-[2-[4-[3-Ethoxy-1 (E)-propenyl]phenyl]-4-[4-(isopropylamino)phenyl]-1 H-imidazol-5-yl]phenyl]-N-isopropylamine; 2-[4-[3-Ethoxy-1(E)-propenyl]phenyl]-4,5-bis[4-(isopropylamino)phenyl]-1 H-imidazole | |
| 56 | Phosphoric acid [(3beta,5alpha,24R)-stigmastan-3-yl] L-ascorbic acid-2-O-yl diester disodium salt; 2-O-[Hydroxy[(3beta,5alpha,24R)-stigmastan-3-O-yl]phosphoryl]-L-ascorbic acid disodium salt | Sitostanol ascorbyl phosphate |
| 57 | N-[4-Methyl-3-[4-(3-pyridyl)pyrimidin-2-ylamino)phenyl]-3,5-bis(trifluoromethyl)benzamide | |
| 58 | 1-(6,7-Dihydro-5H-benzo[6,7]cyclohepta[1,2-c]pyridazin-3-yl)-N3-[7(S)-(1-pyrrolidinyl)-6,7,8,9-tetrahydro-5H-benzocycloheptene-2-yl]-1H-1,2,4-triazole-3,5-diamine | Bemcentinib (USAN; Rec INN) |
| 59 | 5-[5-Phenyl-4-(pyridin-2-ylmethylamino)quinazolin-2-yl]pyridine-3-sulfonamide | |
| 60 | N-[2-(2,1,3-Benzothiadiazol-5-ylamino)-6-(2,6-dichlorophenyl)pyrido[2,3-d]pyrimidin-7-yl]-N'-tert-butylurea | |
| 61 | N-[4-(2-Methyl-1,4,5,6-tetrahydroimidazo[4,5-d][1]benzazepin-6-ylcarbonyl)phenyl]biphenyl-2-carboxamide hydrochloride | Conivaptan hydrochloride (Rec INNM) |
| 62 | N-[4-[3-(N-Benzyl-N-methylaminomethyl)-7-(2,6-difluorobenzyl)-5-isobutyryl-4-oxo-4,7-dihydrothieno[2,3-b]pyridin-2-yl]phenyl]-1-hydroxycyclopropanecarboxamide | |
| 63 | 2-(3-Chloro-4-fluorobenzyl)-8-ethyl-10-hydroxy-6(S)-methyl-4-(5-methyl-1,1-dioxido-1,2,5-thiadiazolidin-2-yl)-1,2,6,7,8,9-hexahydropyrazino[1',2':1,5]pyrrolo[2,3-d]pyridazine-1,9-dione | |
| 64 | (7bR,8aS)-N-[2-[(1,2,4,5,8,8a-Hexahydro-7-methyl-4-oxocyclopropa[c]pyrrolo[3,2-e]indol-2-yl)carbonyl]-1 H-indol-5-yl]-2-benzofu rancarboxamide | Adozelesin (USAN; Rec INN) |
| 65 | N-(2,4-Di-tert-butyl-5-hydroxyphenyl)-4-oxo-1,4-dihydroquinoline-3-carboxamide | Ivacaftor (Prop INN; USAN) |
| 66 | N-(4-tert-Butylphenyl)-N-[2-(cyclohexylamino)-2-oxo-1-(pyridin-3-yl)ethyl]-1 H-imidazole-4-carboxamide | |
| 67 | 1-(3,4-Dimethylphenyl)-3-methyl-4,5-dihydro-1 H-pyrazole-4,5-dione 4-[4-[2-hydroxy-3'-(1 H-tetrazol-5-yl)biphenyl-3-yl]hydrazone choline salt | Totrombopag choline (Prop INNM; USAN) |
| 68 | Piperazine-1 ,4-diylbis[[6-(1 H-benzimidazol-2-yl)pyridin-2-yl]methanone] | |
| 69 | 4,5,6,7-Tetrachloro-2',4',5',7'-tetraiodo-3-oxo-3H-spiro[2-benzofuran-1,9'-xanthene]-3',6'-diolate disodium salt | Rose Bengal disodium |
| 70 | 3-(4-Chlorophenylamino)-10-(4-chlorophenyl)-2,10-dihydro-2-(isopropylimino)phenazine | Clofazimina; Clofazimine (BAN; USAN; Rec INN) |
| 71 | (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide | Avacopan (USAN; Rec INN) |
| 72 | 4-[[(3S,4R,5S)-3-(3-Chloro-2-fluorophenyl)-4-(4-chloro-2-fluorophenyl)-4-cyano-5-(2,2-dimethylpropyl)-D-prolyl]amino]-3-methoxybenzoic acid | Idasanutlin (USAN; Rec INN) |
| 73 | 1-(2-Naphthylmethyl)-3-[(4-nitrophenyl)imino]-2-oxoindoline-5-carboxamide | |
| 74 | N-[3-(7-Methyl-1H-indazol-5-yl)-1-[4-(1-methylpiperidin-4-yl)piperazin-1-yl]-1-oxopropan-2(R)-yl]-4-(2-oxo-1,2-dihydroquinolin-3-yl)piperidine-1-carboxamide | Vazegepant |
| 75 | 2-[2-Fluoro-4-[7-(2-methylpyridin-4-yl)imidazo[1,2-a]pyridin-3-yl]phenyl]-N-[3-(trifluoromethyl)phenyl]acetamide | |
| 76 | N-(2-Fluoro-6-methylphenyl)-6-(4-[[5-methyl-2-(7-oxa-2-azaspiro[3.5]nonan-2-yl)pyridine-3-carbonyl]amino]benzoyl)-5,6-dihydro-4H-thieno[3,2-d][1]benzazepine-2-carboxamide | |
| 77 | 2-[2-[2-[2(E)-[4-(Trifluoromethyl)phenyl]vinyl]-1 H-benzimidazol-5-yl]phenyl]propan-2-ol | Mavatrep (USAN; Rec INN) |
| 78 | 4-Methyl-N-[3-(4-methyl-1H-imidazol-1-yl)-5-(trifluoromethyl)phenyl]-3-[4-(2-pyrazinyl)pyrimidin-2-ylamino]benzamide | Radotinib (Prop INN) |
| 79 | 7-(4,7-Diazaspiro[2.5]octan-7-yl)-2-(2,8-dimethylimidazo[1,2-b]pyridazin-6-yl)-4H-pyrido[1,2-a]pyrimidin-4-one | Risdiplam (USAN; Rec INN) |
| 80 | 3-[(4-Methylphenyl)imino]-1-(2-naphthylmethyl)-2-oxoindoline-5-carboxamide | |
| 81 | 4-(4-Methylpiperazin-1-ylmethyl)-N-[6-methyl-5-[4-(3-pyridyl)pyrimidin-2-ylamino]pyridin-3-yl]-3-(trifluoromethyl)benzamide methanesulfonate | Flumatinib mesylate |
| 82 | 2-(5-Amino-6-oxo-2-phenyl-1,6-dihydropyrimidin-1-yl)-N-[1-(5-tert-butyl-1,3,4-oxadiazol-2-ycarbonyl)-2-methylpropyl]acetamide | Freselestat (Rec INN) |
| 83 | N-[3-[5-(2-Aminopyrimidin-4-yl)-2-tert-butylthiazol-4-yl]-2-fluorophenyl]-2,6-difluorobenzenesulfonamide methanesulfonate | Dabrafenib mesylate (Prop INNM; USAN) |
| 84 | (OC-6-23)-[[3-(Dimethylamino)propyl]dimethylsilinolato-kappaO]hydroxy[29H,31H-phthalocyanato(2-)-kappaN29,kappaN30,kappaN31,kappaN32]silicon | Silicon phthalocyanine 4 |
| 85 | Methanesulfonic acid 2-[3-[4-[(1 H-indazol-5-yl)amino]quinazolin-2-yl]phenoxy]-N-(propan-2-yl)acetamide | |
| 86 | Sodium (2E)-3-[2-[1-[[[2-(5-bromopyrimidin-2-yl)-3-cyclopentyl-1-methyl-1H-indol-6-yl]carbonyl]amino]cyclobutyl]-1-methyl-1H-benzi midazol-6-yl]acrylate | Deleobuvir sodium (USAN; Rec INN) |
| 87 | [4(R),6(S),(E)]-6-[2-[4-(4-Fluorophenyl)-2-isopropyl-6-phenyl-3-pyridyl]vinyl]-4-hydroxytetrahydropyran-2-one | Glenvastatin (Rec INN) |
| 88 | [1-(4-[[4-(4-Hydroxypiperidin-1-yl)phenyl]amino]-5-oxo-5,6-dihydropyrimido[4,5-d]pyridazin-2-yl)piperidin-4-yl]acetonitrile | Gusacitinib (Prop INN; USAN) |
| 89 | N-[2-(Benzylamino)-2-oxo-1-(pyridin-3-yl)ethyl]-N-(4-tert-butylphenyl)-1 H-imidazole-4-carboxamide | |
| 90 | N6-(4,4-Dimethyl-4,5-dihydroisoxazol-2-yl)-N4-[3-methyl-4-([1,2,4]triazolo[1,5-a]pyridin-7-yloxy)phenyl]quinazoline-4,6-diamine | Irbinitinib (former INN); Tucatinib (Prop INN; USAN) |
| 91 | 5-Chloro-N2-[5-methyl-4-(piperidin-4-yl)-2-(propan-2-yloxy)phenyl]-N4-[2-(propan-2-ylsulfonyl)phenyl]pyrimid ine-2,4-diamine | Ceritinib (USAN; Rec INN) |
| 92 | (2S)-tert-Butoxy[4-(2,3-dihydropyrano[4,3,2-de]quinolin-7-yl)-2-methylquinolin-3-yl]ethanoic acid | |
| 93 | cis-3-[8-Amino-1-(2-phenylquinolin-7-yl)imidazo[1,5-a]pyrazin-3-yl]-1-methylcyclobutanol | Linsitinib (Prop INN; USAN) |
| 94 | (Z)-2-[4-[2-[1-[4-[(Propan-2-yl)oxy]phenyl]-1 H-benzimidazol-5-yl]ethenyl]phenyl]propan-2-ol | |
| 95 | 6alpha,9alpha-Difluoro-16alpha,17alpha-[4-(dimethylamino)phenylmethylenedioxy]-11beta-hydroxy-21-(3-benzamido-2-methylpropionyloxy)preqna-1,4-dien-3-one | |
| 96 | N-Cyclopropyl-4-[7-[[(cis-3-hydroxy-3-methylcyclobutyl)methyl]amino]-5-(pyridin-3-yloxy)pyrazolo[1,5-a]pyrimidin-3-yl]-2-methylbenzamide hydrochloride | |
| 97 | (+)-(R)-1-[4-(2-Chloro-5-fluorobenzamido)-3-methoxybenzoyl]-1,2,3,5-tetrahydrospiro[1-benzazepine-4,1'-[2]cyclopentene]-3'-carboxylic acid | |
| 98 | 3-[4-[1-[3-Cyclopentyl-1-methyl-2-(2-pyridyl)-1 H-indol-6-ylcarboxamido]cyclobutylcarboxamido]phenyl]-2-propenoic acid trifluoroacetate | |
| 99 | 6-(1 H-Indazol-6-yl)-N-[4-(morpholin-4-yl)phenyl]imidazo[1,2-a]pyrazin-8-amine dimethanesulfonate | Entospletinib dimesylate (Rec INN) |
| 100 | 2-[(1S)-1-[4-Amino-3-(3-fluoro-4-isopropoxyphenyl)-1H-pyrazolo[3,4-d]pyrimidin-1-yl]ethyl]-6-fluoro-3-(3-fluorophenyl)-4H-chromen-4-one 4-methylbenzenesulfonate | Umbralisib tosylate (Rec INNM; USAN) |
| 101 | 2-Butyl-5-[[11C]-methoxymethyl]-6-(1-oxidopyridin-2-yl)-3-[2'-(1 H-tetrazol-5-yl)biphenyl-4-ylmethyl]-3H-imidazo[4,5-b]pyridine | |
| 102 | 3-Amino-3-methyl-N-[2-oxo-1-[2'-(1 H-tetrazol-5-yl)biphenyl-4-ylmethyl]-2,3,4,5-tetrahydro-1 H-1 - benzazepin-3(R)-yl]butyramide | |
| 103 | 4-([[(1S)-2-[(2E)-3-[3-Chloro-2-fluoro-6-(1 H-tetrazol-1-yl)phenyl]prop-2-enoyl]-5-(4-methyl-2-oxopiperazin-1-yl)-1,2,3,4-tetrahydroisoquinolin-1-yl]carbonyl]amino)benzoic acid hydrochloride | |
| 104 | 5-[2-Hydroxy-3-[(2Z)-2-[3-methyl-5-oxo-1-(5,6,7,8-tetrahydronaphthalen-2-yl)-4,5-dihydro-1 H-pyrazol-4-ylidene]hydrazino]phenyl]furan-2-carboxylic acid bis(2-aminoethanol) | Hetrombopag olamine |
| 105 | 3-[4-(1-Cyclobutylpiperidin-4-yloxy)phenyl]-2-methyl-5-(trifluoromethyl)quinazolin-4(3H)-one | |
| 106 | (R,S)-9-[Benzyloxy(hydroxy)phosphoryloxy]-14-methyl-5,14-dihydrobenz[5,6]isoindolo[2,1-b]isoquinoline-8,13-dione; Benzyl (±)-5,8,13,14-tetrahydro-14-methyl-8,13-dioxobenz[5,6]isoindolo[2,1-b]isoquinolin-9-yl hydrogen phosphate | Fosquidone (BAN; USAN; Rec INN) |
| 107 | 5-[6-[2,4-Bis(trifluoromethyl)phenyl]pyridazin-3-ylmethyl]-2-(2-fluorophenyl)-5H-imidazo[4,5-c]pyridine | Tegobuvir (Prop INN; USAN) |
| 108 | 2,7-Bis[[(3R,5S)-3,5-dimethylpiperidin-1-yl]sulfonyl]-N,N'-dihydroxyanthracene-9,10-diimine | Tegatrabetan; Tegavivint (Rec INN) |
| 109 | 4'-[2-Ethyl-4-methyl-6-(5,6,7,8-tetrahydroimidazo[1,2-a]pyridin-2-yl)benzimidazol-1-ylmethyl]biphenyl-2-carboxylic acid | Pomisartan (Prop INN) |
| 110 | (6S)-N-[(3S,5S,6R)-6-Methyl-2-oxo-1-(2,2,2-trifluoroethyl)-5-(2,3,6-trifluorophenyl)piperidin-3-yl]-2'-oxo-1',2',5, 7-tetrahydrospiro[cyclopenta[b]pyridine-6,3'-pyrrolo[2,3-b]pyridine]-3-carboxamide | Atogepant (Rec INNM; Rec INN) |
| 111 | 5-(4-Cyclopropyl-1 H-imidazol-1-yl)-2-fluoro-N-[6-(4-isopropyl-4H-1,2,4-triazol-3-yl)pyridin-2-yl]-4-methylbenzamide hydrochloride | Selonsertib hydrochloride (USAN; Rec INN) |
| 112 | N-(2-Bromo-3-oxospiro[3.5]non-1-en-1-yl)-4-(2,7-naphthyridin-1-ylamino)-L-phenylalanine ethyl ester | Zaurategrast (Prop INN) |
| 113 | (6aR,9R,10aR)-N-[(2R,5S,10aS,10bS)-10b-Hydroxy-5-isobutyl-2-isopropyl-3,6-dioxoperhydrooxazolo[3,2-a]pyrrolo[1,2-d]pyrazin-2-yl]-7-methyl-4,6,6a,7,8,9,10,10a-octahydroindolo[4,3-fg]quinoline-9-carboxamide mesylate | Dihydro-alpha-ergokryptine mesylate |
| 114 | 4-[9-Chloro-7-(2,6-difluorophenyl)-5H-pyrimido[5,4-d][2]benzazepin-2-ylamino]benzoic acid | |
| 115 | 4-[4-[4-[4-Fluoro-3-(trifluoromethyl)phenyl]-1-methyl-1H-imidazol-2-yl]piperidin-1-yl]-1H-pyrazolo[3,4-d]pyrimidine 4-methylbenzenesulfonate | |
| 116 | Lup-20(29)-ene-3beta,28-diol | Betulin; Betulinic alcohol; Betulinol; Trochol |
| 117 | 1-(2-Naphthylmethyl)-2-oxo-3-(phenylimino)indoline-5-carboxamide | |
| 118 | 3,3'-[(1,6-Dioxo-1,6-hexanediyl)diimino]bis[2,4,6-triiodobenzoic acid] diethyl ester | lodipamide ethyl ester |
| 119 | 2-(Piperidin-1-yl)-N-[4-(trifluoromethyl)phenyl]-7-[3-(trifluoromethyl)pyridin-2-yl]-6,7,8,9-tetrahydro-5H-pyrimido[4,5-d]azepin-4-amine sulfate | |
| 120 | 4,5-Dichloro-N-[3-[1-(2,4-dichlorobenzyl)-5-fluoro-3-methyl-1 H-indol-7-yl]-2(E)-propenoyl]thiophene-2-sulfonamide | |
| 121 | (2E)-N-(4-[[3-Chloro-4-(pyridin-2-ylmethoxy)phenyl]amino]-3-cyano-7-ethoxyquinolin-6-yl)-3-[(2R)-1-methylpyrrolidin-2-yl]prop-2-enamide (2Z)-but-2-enedioate | Pyrotinib maleate |
| 122 | (2S)-N-[(4S,6S,7S)-7-[[(2,6-Dimethyl phenoxy)acetyl]amino]-6-hydroxy-2-methyl-8-phenyloctan-4-yl]-3-methyl-2-(2-oxotetrahydropyrimidin-1(2H)-yl)butanamide | Leucine lopinavir |
| 123 | 4-[2,6-Di-tert-butyl-4-[1-(3,5-di-tert-butyl-4-hydroxyphenylsulfanyl)-1-methylethylsulfanyl]phenoxy]butyric acid | Elsibucol (USAN; Rec INN) |
| 124 | N-[2-(1H-1,2,4-Triazol-5-ylmethyl)-1,2,3,4-tetrahydroisoquinolin-6-yl]-4'-(trifluoromethyl)-1,1'-biphenyl-2-carboxamide | |
| 125 | (1S)-1-(4-Fluorophenyl)-1-(2-[4-[6-(1-methyl-1 H-pyrazol-4-yl)pyrrolo[2,1-f][1,2,4]triazin-4-yl]piperazin-1-yl]pyrimidin-5-yl)ethan-1-amine | Avapritinib (Rec INNM; Rec INN) |
| 126 | 2-Hydroxy-N,N,N-trimethylethan-1-aminium (3beta,18alpha)-3-hydroxy-11-oxoolean-12-en-30-oate | 18alpha-Glycyrrhetinic acid choline salt |
| 127 | N-[4-[3-(2-Aminopyrimidin-4-yl)pyridin-2-yloxy]phenyl]-4-(4-methylthien-2-yl)phthalazin-1-amine | |
| 128 | 2-Hydroxy-1-[2-[[9-(trans-4-methylcyclohexyl)-9H-pyrido[4',3':4,5]pyrrolo[2,3-d]pyrimidin-2-yl]amino]-7,8-dihydro-1,6-naphthyridin-6(5H)-yl]ethanone | |
| 129 | 9-Isopropyl-N2-[4-(4-methylpiperazin-1-yl)phenyl]-N8-(3-pyridyl)-9H-purine-2,8-diamine | |
| 130 | 1(RS)-N-[4-[N-(4-Chlorophenylsulfonyl)carbamoyl]benzoyl]-L-valyl-N-[2-methyl-1-(trifluoroacetyl)propyl]-L-prolinamide | |
| 131 | 2-Amino-5-cyano-6-methyl-4(R)-(3-phenylquinolin-5-yl)-1,4-dihydropyridine-3-carboxylic acid isopropyl ester | |
| 132 | N-[3-tert-Butyl-1-(4-methylphenyl)-1H-pyrazol-5-yl]-N'-[4-[6-(morpholin-4-ylmethyl)pyridin-3-yl]naphthalen-1-yl]urea | |
| 133 | 5-Fluoro-N-[2-[1-methylpyrrolidin-2(R)-yl]ethyl]-3-oxo-6-[3-(2-pyrazinyl)pyrrolidin-1-yl]-3H-benzo[b]pyrido[3,2,1-kl]phenoxazine-2-carboxamide | Itarnafloxin (Prop INN); Quarfloxin (former INN; USAN) |
| 134 | (2R,4aS,6aS,12bR,14aS,14bR)-10-Hydroxy-2,4a,6a,9,12b,14a-hexamethyl-11-oxo-1,2,3,4,4a,5,6,6a,11,12b,13,14,14a,14b-tetradecahydropicene-2-carboxylic acid L-threonine cocrystal | Tripterine threonine |
| 135 | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate | Nelfinavir mesilate (Prop INNM; USAN) |
| 136 | 9-Acetoxy-2-(1,2-dimethylpropyl)-8-(hexopyranosyloxy)-2,4a,7,7,10a,12a-hexamethyl-1,2,3,4,4a,5,6,6a,7,8,9,10,10a,11,12,12a-hexadecahydrochrysene-1-carboxylic acid | |
| 137 | (+)-2-Amino-N-[(1S)-1-[8-[(1-methyl-1H-pyrazol-4-yl)ethynyl]-1-oxo-2-phenyl-1,2-dihydroisoquinolin-3-yl]ethyl]pyrazolo[1,5-a]pyrimidine-3-carboxamide | |
| 138 | 4"'-O-Methyldigoxin; (3beta,5beta,12beta,14beta)-[O-(2,6-Dideoxy-4-O-methyl-D-ribo-hexopyranosyl)-(1--4)-O-(2,6-dideoxy-beta-D-ribo-hexopyranosyl)-(1--4)-2,6-dideoxy-D-ribo-hexopyranosyloxy]-12,14-dihydroxy-card-20(22)-enolide | Medigoxin (BAN); Metildigoxin (USAN; JAN; Rec INN) |
| 139 | N-[2-Fluoro-5-[3-[2-(2-pyridyl)vinyl]-1H-indazol-6-ylamino]phenyl]-1,3-dimethyl-1 H-pyrazole-5-carboxamide | |
| 140 | N-(4-tert-Butylphenyl)-N-[2-(cyclopentylamino)-2-oxo-1 -(pyridin-3-yl)ethyl]-1 H-imidazole-4-carboxamide | |
| 141 | 7-[7-[(3bR,4aS)-3-Methyl-8-oxo-4,4a,5,8-tetrahydrocyclopropa[c]pyrrolo[3,2-e]indol-6(1H)-ylcarbonyl]-4-hydroxy-5-methoxy-1,2,3,6-tetrahydropyrrolo[3,2-e]indol-3-ylcarbonyl]-4-hydroxy-5-methoxy-1,2,3,6-tetrahydropyrrolo[3,2-e]indole-3-carboxamide | Rachelmycin |
| 142 | (6bS,8aS,11R,12aR,12bS,14aR)-3-Hydroxy-4,6b,8a, 11, 12b, 14a-hexamethyl-2,6b,7,8,8a,9,10,11,12,12a,12b,13,14,14a-tetradecahydropicene-2,10-dione | Maytenin; Tingenin A; Tingenone |
| 143 | (aR)-(2S)-6-Fluoro-5-[3-[(aS)-8-fluoro-1-methyl-2,4-dioxo-1,4-dihydroquinazolin-3(2H)-yl]-2-methylphenyl]-2-(2-hydroxypropan-2-yl)-2,3,4,9-tetrahydro-1H-carbazole-8-carboxamide | |
| 144 | 2-Benzyl-5-(4-chlorophenyl)-6-[4-(methylsulfanyl)phenyl]pyridazin-3(2H)-one | |
| 145 | 3-[3-Carboxy-2-methyl-1-[(Z)-5,5,8,8-tetramethyl-5,6,7,8-tetrahydroanthracen-2-ylmethylene]-2(Z)-propenyl]benzoic acid; 4-(3-Carboxyphenyl)-3-methyl-5-(5,5,8,8-tetramethyl-5,6,7,8-tetrahydroanthracen-2-yl)-2(Z),4(Z)-pentadienoic acid | |
| 146 | (S)-N-[1-(2-Fluorophenyl)-4-oxo-3,4,6,7-tetrahydropyrrolo[3,2,1 -jk][1 ,4]benzodiazepin-3-yl]-1 H-indole-2-carboxamide | Pranazepide (Prop INN) |
| 147 | 2',6-Difluoro-5'-[3-(1-hydroxy-1-methylethyl)imidazo[1,2-b][1,2,4]triazin-7-yl]biphenyl-2-carbonitrile | |
| 148 | 4-(Dimethylamino)-N-[3-[4-[2-hydroxy-1(S)-phenylethylamino]-6-phenylfuro[2,3-d]pyrimidin-5-yl]phenyl]-2-butenamide | |
| 149 | N'-[4-[4-(Dimethylamino)piperidin-1-ylcarbonyl]phenyl]-N-[4-[4,6-di(4-morpholinyl)-1,3,5-triazin-2-yl]phenyl]urea | Gedatolisib (USAN; Rec INN) |
| 150 | 6-(2-Chlorophenyl)-9-[(4-methoxyphenyl)carbamoyl]-1-methyl-4,7,8,10-tetrahydropyrido[4',3':4,5]thieno[3,2-f][1,2,4]triazolo[4,3-a][1,4]diazepine | Setipafant (Rec INN) |
| 151 | N-(1',2-Dihydroxy-1,2'-binaphthalen-4'-yl)-4-methoxybenzenesulfonamide | |
| 152 | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid 2-aminoethanol (1:2) | Eltrombopag olamine (Rec INNM; USAN) |
| 153 | (11beta,16beta)-9-Fluoro-1',4'-dihydro-11,21-dihydroxy-2'H-naphtho[2',3':16,17]pregna-1,4-diene-3,20-dione monohydrate; 9-Fluoro-1',4'-dihydro-11beta,21-dihydroxy-2'betaH-naphtho[2',3':16,17]pregna-1,4-diene-3,20-dione monohydrate | Naflocort (BAN; Rec INN) |
| 154 | N-(3-[5-[(1-Ethylpiperidin-4-yl)(methyl)amino]-3-(pyrimidin-5-yl)-1 H-pyrrolo[3,2-b]pyridin-1-yl]-2,4-difluorophenyl)propane-1-sulfonamide | |
| 155 | N-Cycloheptyl-N-(9H-fluoren-2-ylmethyl)-N'-(2,4,6-trimethylphenyl)urea | |
| 156 | 5-[(1 R,3R)-1-(1-Ethyl-1H-pyrazol-4-yl)-3-[4-(5-fluoropyridin-2-yl)-1 H-imidazol-2-yl]-2,3,4,9-tetrahydro-1 H-beta-carbolin-1-yl]-3-methyl-1,3,4-oxadiazol-2(3H)-one | |
| 157 | N-(5-Ethyl-2,4-dimethylphenyl)-4-[3-fluoro-4-(4-methyl-1 H-imidazol-1-yl)phenyl]thiazol-2-amine | |
| 158 | N-[2-(Diethylamino)ethyl]-2,4-dimethyl-5-[2-oxo-5-(2-phenylthiazol-4-yl)-2,3-dihydro-1 H-indol-3-ylidenemethyl]-1 H-pyrrole-3-carboxamide | Amcasertib (USAN; Rec INN) |
| 159 | N-(4-Oxo-3,4-dihydroquinazolin-2-yl)-L-prolyl-N-methyl-L-3-(2-naphthyl)alanine N-benzyl-N-methylamide | |
| 160 | (5S,6R,8R)-3'-(Methylpolyehtyleneglycol)-5-methyl-7,8,14,15-tetrahydro-2'H,4'H,13H-spiro[5,8-epoxy-4b,8a,14-triazadibenzo[b,h]cycloocta[1,2,3,4-jkl]cyclopenta[e]-as-indacene-6,5'-[1,3]oxazolidine]-2',4',13-trione | Pegcantratinib (USAN; Rec INN) |

2. Pharmaceutical composition, for use in the treatment of COVID-19, comprising:
i) a compound inhibitor of 3CL protease of SARS-CoV2 selected from compounds 1 to 160 below:
| **ID** | **Chemical Name** | **Generic Name (GN)** |
|---|---|---|
| 1 | N1-[4-[5-(2-Phenanthryl)-3-(trifluoromethyl)-1H-pyrazol-1-yl]phenyl]glycinamide | |
| 2 | N-(5-[3-[7-(3-Fluorophenyl)-1H-imidazo[4,5-c]pyridin-2-yl]-1 H-indazol-5-yl]pyridin-3-yl)-3-methylbutanamide | Adavivint (former INN); Lorecivivint (USAN; Rec INN) |
| 3 | N-[3-Chloro-4-[(12aS)-3,4,6,11,12,12a-hexahydro-1 H-[1,4]oxazino[3,4-c][1,4]benzodiazepin-11-ylcarbonyl]phenyl]biphenyl-2-carboxamide hydrochloride | |
| 4 | 2-(5-[(Z)-[1-(4-tert-Butylphenyl)-5-oxo-3-phenyl-1,5-dihydro-4H-pyrazol-4-ylidene]methyl]furan-2-yl)-4-chlorobenzoic acid | |
| 5 | 2-[1-(4-tert-Butylbenzyl)-5-(3-methylphenyl)-1 H-indol-3-yl]-2-oxoacetic acid | Aleplasinin (Prop INN; USAN) |
| 6 | N'-[4-[4-[2-[3-(Dimethylaminomethyl)phenyl]-1 H-pyrrolo[2,3-b]pyridin-4-yl]-1-ethyl-1 H-pyrazol-3-yl]phenyl]-N,N-dimethylurea | |
| 7 | (4S,5R)-5-[3,5-Bis(trifluoromethyl)phenyl]-3-([2-[4-fluoro-2-methoxy-5-(propan-2-yl)phenyl]-5,5-dimethylcyclohex-1-en-1-yl]methyl)-4-methyl-1,3-oxazolidin-2-one | Rocacetrapib (Rec INN) |
| 8 | [2-[6-(2-Ethyl-5-fluoro-4-hydroxyphenyl)-1 H-indazol-3-yl]-1 ,4,6,7-tetrahydro-5H-imidazo[4,5-c]pyridin-5-yl][5-(piperidin-1-yl)pyrazin-2-yl]methanone | |
| 9 | 2-Bromoergocryptine monomethanesulfonate | Bromocriptine mesilate (BANM; Rec INNM; USAN; JAN) |
| 10 | (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester; (+)-(R)-4-(2-Chlorophenyl)-6-methyl-2-[4-(2-methylimidazo[4,5-c]pyridin-1-yl)phenyl]-5-(2-pyridylcarbamoyl)-1,4-dihydronicotinic acid ethyl ester | Modipafant (BAN; Rec INN) |
| 11 | 4-(2-Chlorophenyl)-6-methyl-2-[4-(2-methylimidazo[4,5-c]pyridin-1-yl)phenyl]-5-(2-pyridylcarbamoyl)-1,4-dihydropyridine-3-carboxylic acid ethyl ester | |
| 12 | 4-Methyl-N-[3-(4-methyl-1H-imidazol-1-yl)-5-(trifluoromethyl)phenyl]-3-[4-(3-pyridyl)pyrimidin-2-ylamino]benzamide hydrochloride monohydrate | Nilotinib hydrochloride monohydrate (Rec INNM; USAN) |
| 13 | 4-Chloro-2-[5-[[1-(4-fluorophenyl)-5-oxo-3-phenyl-1,5-dihydro-4H-pyrazol-4-ylidene]methyl]-2-furyl]benzoic acid | |
| 14 | 2-Chloro-6-methyl-N'-[[4-methyl-3-(quinolin-3-ylethynyl)phenyl]carbonyl]benzohydrazide | |
| 15 | (±)-N-(4-Amino-3,4-dioxo-1-phenylbutan-2-yl)-2-[3-(4-fluorophenyl)-1 H-pyrazol-1-yl]pyridine-3-carboxamide | |
| 16 | 3(R)-(2,3-Dihydro-1-benzofuran-5-yl)-2-[5-(2-pyridyl)pyrimidin-2-yl]-2,3,4,9-tetrahydro-1H-pyrrolo[3,4-b]quinolin-9-one | |
| 17 | 4-Chloro-2-[5-[(5-oxo-1,3-diphenyl-1,5-dihydro-4H-pyrazol-4-ylidene)methyl]-2-furyl]benzoic acid | |
| 18 | 4-Amino-N-[1-(3-chloro-2-fluoroanilino)-6-methylisoquinolin-5-yl]thieno[3,2-d]pyrimidine-7-carboxamide | Belvarafenib (Rec INN) |
| 19 | (S,S)-6,6'-[Carbonylbis(5-imino-1 H-indole-2-carbonyl)]bis[8-(chloromethyl)-1-methyl-3,6,7,8-tetrahydrobenzo[1,2-b:4,3-b']dipyrrol-4-ol]; (S,S)-1,3-Bis[2-[1-(Chloromethyl)-5-hydroxy-8-methyl-1,2,3,6-tetrahydrobenzo[1,2-b:4,3-b']dipyrrol-3-ylcarbonyl]-1 H-indol-5-yl]urea | Bizelesin (USAN; Rec INN) |
| 20 | N-[3-[6-[4-[1,4-Dimethyl-3-oxopiperazin-2(R)-yl]phenylamino]-4-methyl-5-oxo-4,5-dihydropyrazin-2-yl]-2-methylphenyl]-4,5,6,7-tetrahydro-1-benzothiophene-2-carboxamide | |
| 21 | 2-Hydroxy-5-[4-[3-[4-(2-methylimidazo[4,5-c]pyridin-1-ylmethyl)piperidin-1-yl]-3-oxo-1-phenyl-1(Z)-propenyl]phenylazo]benzoic acid | Dersalazine (Rec INN) |
| 22 | (-)-2-[3-(1,4-Dimethyl-1 H-1,2,3-triazol-5-yl)-5-[(S)-phenyl(tetrahydro-2H-pyran-4-yl)methyl]-5H-pyrido[3,2-b]indol-7-yl]propan-2-ol | |
| 23 | 4-[(2R,4R)-4-[[1-(2,2-Difluoro-2H-1,3-benzodioxol-5-yl)cyclopropane-1-carbonyl]amino]-7-(difluoromethoxy)-3,4-dihydro-2H-1-benzopyran-2-yl]benzoic acid | Galicaftor (USAN; Rec INN) |
| 24 | 2-[5-[(5-Oxo-1,3-diphenyl-1,5-dihydro-4H-pyrazol-4-ylidene)methyl]furan-2-yl]benzoic acid | |
| 25 | 1-[2-[1-[3,5-Bis(trifluoromethyl)benzyl]-5-(4-pyridyl)-1H-1,2,3-triazol-4-yl]pyridin-3-yl]-1-(2-chlorophenyl)methanone | Tradipitant (Prop INN; USAN) |
| 26 | 6-[(5S,9R)-9-(4-Cyanophenyl)-3-(3,5-dichlorophenyl)-1-methyl-2,4-dioxo-1,3,7-triazaspiro[4.4]non-7-yl]pyridine-3-carboxylic acid | |
| 27 | (6S)-6-(2-Hydroxy-2-methylpropyl)-3-[(1S)-1-[4-(1-methyl-2-oxo-1,2-dihydropyridin-4-yl)phenyl]ethyl]-6-phenyltetrahydro-2H-1,3-oxazin-2-one hydrate | |
| 28 | Carbonic acid cholest-5-en-3beta-yl 3,5-dichloro-2-methoxy-6-(trichloromethyl)pyridin-4-yl diester | 4-Demethyl-4-cholesteryloxycarbonylpenc lomedine |
| 29 | 3-[3-[1-(N-Isopropyl-N-phenylcarbamoylmethyl)-2,4-dioxo-5-phenyl-2,3,4,5-tetrahydro-1H-1,5-benzodiazepin-3(S)-yl]ureido]benzoic acid | |
| 30 | 2-[3-[N-Methyl-N-[1-(naphthalen-2-ylcarbonyl)piperidin-4-yl]carbamoyl]naphthalen-2-yl]-1-(1-naphthyl)-2-oxoethylphosphonic acid | |
| 31 | 2-Propyl-1-[2'-(1 H-tetrazol-5-yl)biphenyl-4-ylmethyl]-4-[2-(trifluoroacetyl)pyrrol-1-yl]imidazole-5-carboxylic acid | |
| 32 | 4'-[4-Methyl-6-(1-methyl-1H-benzimidazol-2-yl)-2-propyl-1H-benzimidazol-1-ylmethyl]biphenyl-2-carboxylic acid | Telmisartan (USAN; Rec INN) |
| 33 | 5-Chloro-N-(3-cyclopropyl-5-[(cis-3,5-dimethylpiperazin-1-yl)methyl]phenyl)-4-(6-methyl-1 H-indol-3-yl)pyrimidin-2-amine hydrate | |
| 34 | 11-Keto-beta-boswellic acid; 3alpha-Hydroxyurs-12-en-11-keto-23-oic acid | 11-Keto-beta-boswellic acid |
| 35 | N-(4-tert-Butylphenyl)-N-[2-[(2-methylpropyl)amino]-2-oxo-1-(pyridin-3-yl)ethyl]-1 H-imidazole-4-carboxamide | |
| 36 | 5-[N-[5-[N-(Cycloheptylmethyl)carbamoyl]-1 H-benzimidazol-6-ylcarbonyl]-2-fluoro-L-phenylalanylamino]isophthalic acid; 5-[N-[5-[N-(Cycloheptylmethyl)carbamoyl]-1 H-benzimidazol-6-ylcarbonyl]-2-fluoro-L-phenylalanylamino]benzene-1,3-dicarboxylic acid | Gastrazole |
| 37 | 21-[4-[2,6-Bis(pyrrolidino)pyrimidin-4-yl]piperazin-1-yl]-16alpha-methyl-pregna-1,4,9(11)-triene-3,20-dione methanesulfonate hydrate; 16alpha-Methyl-21-[4-[2,6-bis(pyrrolidino)-4-pyrimidinyl]-1-piperazinyl]pregna-1,4,9(11 )triene-3,20-dione monomethanesulfonate hydrate | Tirilazad mesylate (Rec INNM; BAN; USAN) |
| 38 | 3-[5-Oxo-3-phenyl-4-[(5-phenylfuran-2-yl)methylidene]-4,5-dihydro-1 H-pyrazol-1-yl]benzoic acid | |
| 39 | 2-[2-[2-(2-Chlorophenyl)propan-2-yl]-1-[3'-(methylsulfonyl)biphenyl-4-yl]-1H-imidazol-4-yl]propan-2-ol | |
| 40 | 4-[4-[3-[3-tert-Butyl-1-(6-quinolinyl)-1H-pyrazol-5-yl]ureido]-3-fluorophenoxy]-N-methylpyridine-2-carboxamide tosylate salt | Rebastinib tosylate (USAN; Rec INN) |
| 41 | Benzoic acid 4-chloro-3-[5-methyl-3-[4-[2-(1-pyrrolidinyl)ethoxylphenylamino]-1,2,4-benzotriazin-7-yl]phenyl ester | |
| 42 | N-[3-Fluoro-4-[[11 C]methyloxy]phenyl]-6-[(2R)-2-(3-fluorophenyl)pyrrolidin-1-yl]imidazo[1,2-b]pyridazine-3-carboxamide | |
| 43 | 4-Fluoro-2-(4-fluorophenyl)-N-methyl-5-(2-methyl-5-[[1-(pyrimidin-2-yl)cyclopropyl]carbamoyl]phenyl)-1-benzofuran-3-carboxamide | |
| 44 | 6-Methyl-N5-[3-(1 H-purin-6-yl)pyridin-2-yl]-N1-[3-(trifluoromethoxy)phenyl]isoquinoline-1,5-diamine | |
| 45 | (aR)-6-Fluoro-7-(2-fluoro-6-hydroxyphenyl)-1-[4-methyl-2-(propan-2-yl)pyridin-3-yl]-4-[(2S)-2-methyl-4-(prop-2-enoyl)piperazin-1-yl]pyrido[2,3-d]pyrimidin-2(1 H)-one | |
| 46 | N-(3-Fluoro-4-[[7-(2-hydroxy-2-methylpropoxy)quinolin-4-yl]oxy]phenyl)-1,5-dimethyl-3-oxo-2-phenyl-2,3-dihydro-1 H-pyrazole-4-carboxamide 4-methylbenzenesulfonate | Ningetinib Tosylate; Ningetinib (free base) |
| 47 | 4-[2-[4-(4-Ethylphenyl)-2,2-dimethyl-2H-1-benzothiopyran-6-yl]ethynyl]benzoic acid | |
| 48 | (2E)-3-(6-Aminopyridin-3-yl)-N-([5-[4-(4,4-difluoropiperidine-1-carbonyl)phenyl]-7-(4-fluorophenyl)-1-benzofuran-2-yl]methyl)prop-2-enamide | |
| 49 | 3-Chloro-N-[1-(N,N-dimethylglycinamido)-3-[4-[8-[1(S)-hydroxyethyl]imidazo[1,2-a]pyridin-2-yl]phenyl]propan-2(S)-yl]-4-isopropoxybenzamide | |
| 50 | Phosphoric acid (6aR,9aS)-3-anilino-2-[[4-(6-fluoropyridin-2-yl)phenyl]methyl]-5-methyl-5,6a,7,8,9,9a-hexahydrocyclopenta[4,5]imidazo[1,2-a]pyrazolo[4,3-e]pyrimidin-4(2H)-one | |
| 51 | (4R)-1-[[3-Acetyl-5-(2-methylpyrimidin-5-yl)-1H-indazol-1-yl]acetyl]-N-(6-bromopyridin-2-yl)-4-fluoro-L-prolinamide | Danicopan (USAN; Rec INN) |
| 52 | 1-[3-Bromo-2-[2-(trifluoromethylsulfonamido)phenyl]benzofuran-5-ylmethyl]-4-cyclopropyl-2-ethyl-1H-imidazole-5-carboxamide | Saprisartan (BAN; USAN; Rec INN) |
| 53 | 2-(4-Fluorophenyl)-5-(11-fluoro-6H-pyrido[2',3':5,6][1,3]oxazino[3,4-a]indol-2-yl)-6-[(methanesulfonyl)(methyl)amino]-N-methyl-1-benzofu ran-3-carboxamide | |
| 54 | Bis(4,4'-dimethyl-2,2'-bipyridine) 2-[[1(2),2(2):2(5),3(2)-terthiophen]-1(5)-yl]-1 H-imidazo[4,5-f][1,10]phenanthroline dichloridoruthenium | |
| 55 | N-[4-[2-[4-[3-Ethoxy-1 (E)-propenyl]phenyl]-4-[4-(isopropylamino)phenyl]-1 H-imidazol-5-yl]phenyl]-N-isopropylamine; 2-[4-[3-Ethoxy-1(E)-propenyl]phenyl]-4,5-bis[4-(isopropylamino)phenyl]-1 H-imidazole | |
| 56 | Phosphoric acid [(3beta,5alpha,24R)-stigmastan-3-yl] L-ascorbic acid-2-O-yl diester disodium salt; 2-O-[Hydroxy[(3beta,5alpha,24R)-stigmastan-3-O-yl]phosphoryl]-L-ascorbic acid disodium salt | Sitostanol ascorbyl phosphate |
| 57 | N-[4-Methyl-3-[4-(3-pyridyl)pyrimidin-2-ylamino)phenyl]-3,5-bis(trifluoromethyl)benzamide | |
| 58 | 1-(6,7-Dihydro-5H-benzo[6,7]cyclohepta[1,2-c]pyridazin-3-yl)-N3-[7(S)-(1-pyrrolidinyl)-6,7,8,9-tetrahydro-5H-benzocycloheptene-2-yl]-1H-1,2,4-triazole-3,5-diamine | Bemcentinib (USAN; Rec INN) |
| 59 | 5-[5-Phenyl-4-(pyridin-2-ylmethylamino)quinazolin-2-yl]pyridine-3-sulfonamide | |
| 60 | N-[2-(2,1,3-Benzothiadiazol-5-ylamino)-6-(2,6-dichlorophenyl)pyrido[2,3-d]pyrimidin-7-yl]-N'-tert-butyl urea | |
| 61 | N-[4-(2-Methyl-1,4,5,6-tetrahydroimidazo[4,5-d][1]benzazepin-6-ylcarbonyl)phenyl]biphenyl-2-carboxamide hydrochloride | Conivaptan hydrochloride (Rec INNM) |
| 62 | N-[4-[3-(N-Benzyl-N-methylaminomethyl)-7-(2,6-difluorobenzyl)-5-isobutyryl-4-oxo-4,7-dihydrothieno[2,3-b]pyridin-2-yl]phenyl]-1-hydroxycyclopropanecarboxamide | |
| 63 | 2-(3-Chloro-4-fluorobenzyl)-8-ethyl-10-hydroxy-6(S)-methyl-4-(5-methyl-1,1-dioxido-1,2,5-thiadiazolidin-2-yl)-1,2,6,7,8,9-hexahydropyrazino[1',2':1,5]pyrrolo[2,3-d]pyridazine-1,9-dione | |
| 64 | (7bR,8aS)-N-[2-[(1,2,4,5,8,8a-Hexahydro-7-methyl-4-oxocyclopropa[c]pyrrolo[3,2-e]indol-2-yl)carbonyl]-1 H-indol-5-yl]-2-benzofu rancarboxamide | Adozelesin (USAN; Rec INN) |
| 65 | N-(2,4-Di-tert-butyl-5-hydroxyphenyl)-4-oxo-1,4-dihydroquinoline-3-carboxamide | Ivacaftor (Prop INN; USAN) |
| 66 | N-(4-tert-Butylphenyl)-N-[2-(cyclohexylamino)-2-oxo-1 -(pyridin-3-yl)ethyl]-1 H-imidazole-4-carboxamide | |
| 67 | 1-(3,4-Dimethylphenyl)-3-methyl-4,5-dihydro-1 H-pyrazole-4,5-dione 4-[4-[2-hydroxy-3'-(1 H-tetrazol-5-yl)biphenyl-3-yl]hydrazone choline salt | Totrombopag choline (Prop INNM; USAN) |
| 68 | Piperazine-1 ,4-diylbis[[6-(1 H-benzimidazol-2-yl)pyridin-2-yl]methanone] | |
| 69 | 4,5,6,7-Tetrach!oro-2',4',5',7'-tetraiodo-3-oxo-3H-spiro[2-benzofuran-1,9'-xanthene]-3',6'-diolate disodium salt | Rose Bengal disodium |
| 70 | 3-(4-Chlorophenylamino)-10-(4-chlorophenyl)-2,10-dihydro-2-(isopropylimino)phenazine | Clofazimina; Clofazimine (BAN; USAN; Rec INN) |
| 71 | (2R,3S)-2-[4-(Cyclopentylamino)phenyl]-1-(2-fluoro-6-methylbenzoyl)-N-[4-methyl-3-(trifluoromethyl)phenyl]piperidine-3-carboxamide | Avacopan (USAN; Rec INN) |
| 72 | 4-[[(3S,4R,5S)-3-(3-Chloro-2-fluorophenyl)-4-(4-chloro-2-fluorophenyl)-4-cyano-5-(2,2-dimethylpropyl)-D-prolyl]amino]-3-methoxybenzoic acid | Idasanutlin (USAN; Rec INN) |
| 73 | 1-(2-Naphthylmethyl)-3-[(4-nitrophenyl)imino]-2-oxoindoline-5-carboxamide | |
| 74 | N-[3-(7-Methyl-1H-indazol-5-yl)-1-[4-(1-methylpiperidin-4-yl)piperazin-1-yl]-1-oxopropan-2(R)-yl]-4-(2-oxo-1,2-dihydroquinolin-3-yl)piperidine-1-carboxamide | Vazegepant |
| 75 | 2-[2-Fluoro-4-[7-(2-methylpyridin-4-yl)imidazo[1,2-a]pyridin-3-yl]phenyl]-N-[3-(trifluoromethyl)phenyl]acetamide | |
| 76 | N-(2-Fluoro-6-methylphenyl)-6-(4-[[5-methyl-2-(7-oxa-2-azaspiro[3.5]nonan-2-yl)pyridine-3-carbonyl]amino]benzoyl)-5,6-dihydro-4H-thieno[3,2-d][1]benzazepine-2-carboxamide | |
| 77 | 2-[2-[2-[2(E)-[4-(Trifluoromethyl)phenyl]vinyl]-1 H-benzimidazol-5-yl]phenyl]propan-2-ol | Mavatrep (USAN; Rec INN) |
| 78 | 4-Methyl-N-[3-(4-methyl-1H-imidazol-1-yl)-5-(trifluoromethyl)phenyl]-3-[4-(2-pyrazinyl)pyrimidin-2-ylamino]benzamide | Radotinib (Prop INN) |
| 79 | 7-(4,7-Diazaspiro[2.5]octan-7-yl)-2-(2,8-dimethylimidazo[1,2-b]pyridazin-6-yl)-4H-pyrido[1,2-a]pyrimidin-4-one | Risdiplam (USAN; Rec INN) |
| 80 | 3-[(4-Methylphenyl)imino]-1-(2-naphthylmethyl)-2-oxoindoline-5-carboxamide | |
| 81 | 4-(4-Methylpiperazin-1-ylmethyl)-N-[6-methyl-5-[4-(3-pyridyl)pyrimidin-2-ylamino]pyridin-3-yl]-3-(trifluoromethyl)benzamide methanesulfonate | Flumatinib mesylate |
| 82 | 2-(5-Amino-6-oxo-2-phenyl-1,6-dihydropyrimidin-1 - yl)-N-[1-(5-tert-butyl-1,3,4-oxadiazol-2-ycarbonyl)-2-methylpropyl]acetamide | Freselestat (Rec INN) |
| 83 | N-[3-[5-(2-Aminopyrimidin-4-yl)-2-tert-butylthiazol-4-yl]-2-fluorophenyl]-2,6-difluorobenzenesulfonamide methanesulfonate | Dabrafenib mesylate (Prop INNM; USAN) |
| 84 | (OC-6-23)-[[3-(Dimethylamino)propyl]dimethylsilinolato-kappaO]hydroxy[29H,31H-phthalocyanato(2-)-kappaN29,kappaN30,kappaN31,kappaN32]silicon | Silicon phthalocyanine 4 |
| 85 | Methanesulfonic acid 2-[3-[4-[(1 H-indazol-5-yl)amino]quinazolin-2-yl]phenoxy]-N-(propan-2-yl)acetamide | |
| 86 | Sodium (2E)-3-[2-[1-[[[2-(5-bromopyrimidin-2-yl)-3-cyclopentyl-1-methyl-1H-indol-6-yl]carbonyl]amino]cyclobutyl]-1-methyl-1H-benzimidazol-6-yl]acrylate | Deleobuvir sodium (USAN; Rec INN) |
| 87 | [4(R),6(S),(E)]-6-[2-[4-(4-Fluorophenyl)-2-isopropyl-6-phenyl-3-pyridyl]vinyl]-4-hydroxytetrahydropyran-2-one | Glenvastatin (Rec INN) |
| 88 | [1-(4-[[4-(4-Hydroxypiperidin-1-yl)phenyl]amino]-5-oxo-5,6-dihydropyrimido[4,5-d]pyridazin-2-yl)piperidin-4-yl]acetonitrile | Gusacitinib (Prop INN; USAN) |
| 89 | N-[2-(Benzylamino)-2-oxo-1-(pyridin-3-yl)ethyl]-N-(4-tert-butylphenyl)-1 H-imidazole-4-carboxamide | |
| 90 | N6-(4,4-Dimethyl-4,5-dihydroisoxazol-2-yl)-N4-[3-methyl-4-([1,2,4]triazolo[1,5-a]pyridin-7-yloxy)phenyl]quinazoline-4,6-diamine | Irbinitinib (former INN); Tucatinib (Prop INN; USAN) |
| 91 | 5-Chloro-N2-[5-methyl-4-(piperidin-4-yl)-2-(propan-2-yloxy)phenyl]-N4-[2-(propan-2-ylsulfonyl)phenyl]pyrimidine-2,4-diamine | Ceritinib (USAN; Rec INN) |
| 92 | (2S)-tert-Butoxy[4-(2,3-dihydropyrano[4,3,2-de]quinolin-7-yl)-2-methylquinolin-3-yl]ethanoic acid | |
| 93 | cis-3-[8-Amino-1-(2-phenylquinolin-7-yl)imidazo[1,5-a]pyrazin-3-yl]-1-methylcyclobutanol | Linsitinib (Prop INN; USAN) |
| 94 | (Z)-2-[4-[2-[1-[4-[(Propan-2-yl)oxy]phenyl]-1 H-benzimidazol-5-yl]ethenyl]phenyl]propan-2-ol | |
| 95 | 6alpha,9alpha-Difluoro-16alpha,17alpha-[4-(dimethylamino)phenylmethylenedioxy]-11beta-hydroxy-21-(3-benzamido-2-methylpropionyloxy)pregna-1,4-dien-3-one | |
| 96 | N-Cyclopropyl-4-[7-[[(cis-3-hydroxy-3-methylcyclobutyl)methyl]amino]-5-(pyridin-3-yloxy)pyrazolo[1,5-a]pyrimidin-3-yl]-2-methylbenzamide hydrochloride | |
| 97 | (+)-(R)-1-[4-(2-Chloro-5-fluorobenzamido)-3-methoxybenzoyl]-1,2,3,5-tetrahydrospiro[1-benzazepine-4,1'-[2]cyclopentene]-3'-carboxylic acid | |
| 98 | 3-[4-[1-[3-Cyclopentyl-1-methyl-2-(2-pyridyl)-1 H-indol-6-ylcarboxamido]cyclobutylcarboxamido]phenyl]-2-propenoic acid trifluoroacetate | |
| 99 | 6-(1 H-Indazol-6-yl)-N-[4-(morpholin-4-yl)phenyl]imidazo[1,2-a]pyrazin-8-amine dimethanesulfonate | Entospletinib dimesylate (Rec INN) |
| 100 | 2-[(1S)-1-[4-Amino-3-(3-fluoro-4-isopropoxyphenyl)-1H-pyrazolo[3,4-d]pyrimidin-1-yl]ethyl]-6-fluoro-3-(3-fluorophenyl)-4H-chromen-4-one 4-methylbenzenesulfonate | Umbralisib tosylate (Rec INNM; USAN) |
| 101 | 2-Butyl-5-[[11C]-methoxymethyl]-6-(1-oxidopyridin-2-yl)-3-[2'-(1 H-tetrazol-5-yl)biphenyl-4-ylmethyl]-3H-imidazo[4,5-b]pyridine | |
| 102 | 3-Amino-3-methyl-N-[2-oxo-1-[2'-(1 H-tetrazol-5-yl)biphenyl-4-ylmethyl]-2,3,4,5-tetrahydro-1 H-1 - benzazepin-3(R)-yl]butyramide | |
| 103 | 4-([[(1S)-2-[(2E)-3-[3-Chloro-2-fluoro-6-(1 H-tetrazol-1-yl)phenyl]prop-2-enoyl]-5-(4-methyl-2-oxopiperazin-1-yl)-1,2,3,4-tetrahydroisoquinolin-1-yl]carbonyl]amino)benzoic acid hydrochloride | |
| 104 | 5-[2-Hydroxy-3-[(2Z)-2-[3-methyl-5-oxo-1-(5,6,7,8-tetrahydronaphthalen-2-yl)-4,5-dihydro-1 H-pyrazol-4-ylidene]hydrazino]phenyl]furan-2-carboxylic acid bis(2-aminoethanol) | Hetrombopag olamine |
| 105 | 3-[4-(1-Cyclobutylpiperidin-4-yloxy)phenyl]-2-methyl-5-(trifluoromethyl)quinazolin-4(3H)-one | |
| 106 | (R,S)-9-[Benzyloxy(hydroxy)phosphoryloxy]-14-methyl-5,14-dihydrobenz[5,6]isoindolo[2,1-b]isoquinoline-8,13-dione; Benzyl (±)-5,8,13,14-tetrahydro-14-methyl-8,13-dioxobenz[5,6]isoindolo[2,1-b]isoquinolin-9-yl hydrogen phosphate | Fosquidone (BAN; USAN; Rec INN) |
| 107 | 5-[6-[2,4-Bis(trifluoromethyl)phenyl]pyridazin-3-ylmethyl]-2-(2-fluorophenyl)-5H-imidazo[4,5-c]pyridine | Tegobuvir (Prop INN; USAN) |
| 108 | 2,7-Bis[[(3R,5S)-3,5-dimethylpiperidin-1-yl]sulfonyl]-N,N'-dihydroxyanthracene-9,10-diimine | Tegatrabetan; Tegavivint (Rec INN) |
| 109 | 4'-[2-Ethyl-4-methyl-6-(5,6,7,8-tetrahydroimidazo[1,2-a]pyridin-2-yl)benzimidazol-1-ylmethyl]biphenyl-2-carboxylic acid | Pomisartan (Prop INN) |
| 110 | (6S)-N-[(3S,5S,6R)-6-Methyl-2-oxo-1-(2,2,2-trifluoroethyl)-5-(2,3,6-trifluorophenyl)piperidin-3-yl]-2'-oxo-1',2',5,7-tetrahydrospiro[cyclopenta[b]pyridine-6,3'-pyrrolo[2,3-b]pyridine]-3-carboxamide | Atogepant (Rec INNM; Rec INN) |
| 111 | 5-(4-Cyclopropyl-1 H-imidazol-1-yl)-2-fluoro-N-[6-(4-isopropyl-4H-1,2,4-triazol-3-yl)pyridin-2-yl]-4-methylbenzamide hydrochloride | Selonsertib hydrochloride (USAN; Rec INN) |
| 112 | N-(2-Bromo-3-oxospiro[3.5]non-1-en-1-yl)-4-(2,7-naphthyridin-1-ylamino)-L-phenylalanine ethyl ester | Zaurategrast (Prop INN) |
| 113 | (6aR,9R,10aR)-N-[(2R,5S,10aS,10bS)-10b-Hydroxy-5-isobutyl-2-isopropyl-3,6-dioxoperhydrooxazolo[3,2-a]pyrrolo[1,2-d]pyrazin-2-yl]-7-methyl-4,6,6a,7,8,9,10,10a-octahydroindolo[4,3-fg]quinoline-9-carboxamide mesylate | Dihydro-alpha-ergokryptine mesylate |
| 114 | 4-[9-Chloro-7-(2,6-difluorophenyl)-5H-pyrimido[5,4-d][2]benzazepin-2-ylamino]benzoic acid | |
| 115 | 4-[4-[4-[4-Fluoro-3-(trifluoromethyl)phenyl]-1-methyl-1H-imidazol-2-yl]piperidin-1-yl]-1H-pyrazolo[3,4-d]pyrimidine 4-methylbenzenesulfonate | |
| 116 | Lup-20(29)-ene-3beta,28-diol | Betulin; Betulinic alcohol; Betulinol; Trochol |
| 117 | 1-(2-Naphthylmethyl)-2-oxo-3-(phenylimino)indoline-5-carboxamide | |
| 118 | 3,3'-[(1,6-Dioxo-1,6-hexanediyl)diimino]bis[2,4,6-triiodobenzoicacid] diethyl ester | Iodipamide ethyl ester |
| 119 | 2-(Piperidin-1-yl)-N-[4-(trifluoromethyl)phenyl]-7-[3-(trifluoromethyl)pyridin-2-yl]-6,7,8,9-tetrahydro-5H-pyrimido[4,5-d]azepin-4-amine sulfate | |
| 120 | 4,5-Dichloro-N-[3-[1-(2,4-dichlorobenzyl)-5-fluoro-3-methyl-1 H-indol-7-yl]-2(E)-propenoyl]thiophene-2-sulfonamide | |
| 121 | (2E)-N-(4-[[3-Chloro-4-(pyridin-2-ylmethoxy)phenyl]amino]-3-cyano-7-ethoxyquinolin-6-yl)-3-[(2R)-1-methylpyrrolidin-2-yl]prop-2-enamide (2Z)-but-2-enedioate | Pyrotinib maleate |
| 122 | (2S)-N-[(4S,6S,7S)-7-[[(2,6-Di methyl phenoxy)acetyl]amino]-6-hydroxy-2-methyl-8-phenyloctan-4-yl]-3-methyl-2-(2-oxotetrahydropyrimidin-1(2H)-yl)butanamide | Leucine lopinavir |
| 123 | 4-[2,6-Di-tert-butyl-4-[1-(3,5-di-tert-butyl-4-hydroxyphenylsulfanyl)-1-methylethylsulfanyl]phenoxy]butyric acid | Elsibucol (USAN; Rec INN) |
| 124 | N-[2-(1H-1,2,4-Triazol-5-ylmethyl)-1,2,3,4-tetrahydroisoquinolin-6-yl]-4'-(trifluoromethyl)-1,1'-biphenyl-2-carboxamide | |
| 125 | (1S)-1-(4-Fluorophenyl)-1-(2-[4-[6-(1-methyl-1 H-pyrazol-4-yl)pyrrolo[2,1 -f][1 ,2,4]triazin-4-yl]piperazin-1-yl]pyrimidin-5-yl)ethan-1-amine | Avapritinib (Rec INNM; Rec INN) |
| 126 | 2-Hydroxy-N,N,N-trimethylethan-1-aminium (3beta,18alpha)-3-hydroxy-11 -oxoolean-12-en-30-oate | 18alpha-Glycyrrhetinic acid choline salt |
| 127 | N-[4-[3-(2-Aminopyrimidin-4-yl)pyridin-2-yloxy]phenyl]-4-(4-methylthien-2-yl)phthalazin-1-amine | |
| 128 | 2-Hydroxy-1-[2-[[9-(trans-4-methylcyclohexyl)-9H-pyrido[4',3^{,}:4,5]pyrrolo[2,3-d]pyrimidin-2-yl]amino]-7,8-dihydro-1,6-naphthyridin-6(5H)-yl]ethanone | |
| 129 | 9-Isopropyl-N2-[4-(4-methylpiperazin-1-yl)phenyl]-N8-(3-pyridyl)-9H-purine-2,8-diamine | |
| 130 | 1(RS)-N-[4-[N-(4-Chlorophenylsulfonyl)carbamoyl]benzoyl]-L-valyl-N-[2-methyl-1-(trifluoroacetyl)propyl]-L-prolinamide | |
| 131 | 2-Amino-5-cyano-6-methyl-4(R)-(3-phenylquinolin-5-yl)-1,4-dihydropyridine-3-carboxylic acid isopropyl ester | |
| 132 | N-[3-tert-Butyl-1-(4-methylphenyl)-1H-pyrazol-5-yl]-N'-[4-[6-(morpholin-4-ylmethyl)pyridin-3-yl]naphthalen-1-yl]urea | |
| 133 | 5-Fluoro-N-[2-[1-methylpyrrolidin-2(R)-yl]ethyl]-3-oxo-6-[3-(2-pyrazinyl)pyrrolidin-1-yl]-3H-benzo[b]pyrido[3,2,1-kl]phenoxazine-2-carboxamide | Itarnafloxin (Prop INN); Quarfloxin (former INN; USAN) |
| 134 | (2R,4aS,6aS,12bR,14aS,14bR)-10-Hydroxy-2,4a,6a,9,12b,14a-hexamethyl-11-oxo-1,2,3,4,4a,5,6,6a,11,12b,13,14,14a,14b-tetradecahydropicene-2-carboxylic acid L-threonine cocrystal | Tripterine threonine |
| 135 | (3S,4aS,8aS)-N-tert-Butyl-2-[2(R)-hydroxy-3(R)-(3-hydroxy-2-methylbenzamido)-4-(phenylsulfanyl)butyl]perhydroisoquinoline-3-carboxamide methanesulfonate | Nelfinavir mesilate (Prop INNM; USAN) |
| 136 | 9-Acetoxy-2-(1,2-dimethylpropyl)-8-(hexopyranosyloxy)-2,4a,7,7,10a,12a-hexamethyl-1,2,3,4,4a,5,6,6a,7,8,9,10,10a,11,12,12a-hexadecahydrochrysene-1-carboxylic acid | |
| 137 | (+)-2-Amino-N-[(1S)-1-[8-[(1-methyl-1 H-pyrazol-4-yl)ethynyl]-1-oxo-2-phenyl-1,2-dihydroisoquinolin-3-yl]ethyl]pyrazolo[1,5-a]pyrimidine-3-carboxamide | |
| 138 | 4"'-O-Methyldigoxin; (3beta,5beta,12beta,14beta)-[O-(2,6-Dideoxy-4-O-methyl-D-ribo-hexopyranosyl)-(1--4)-O-(2,6-dideoxy-beta-O-ribo-hexopyranosyl)-(1--4)-2,6-dideoxy-D-ribo-hexopyranosyloxy]-12,14-dihydroxy-card-20(22)-enolide | Medigoxin (BAN); Metildigoxin (USAN; JAN; Rec INN) |
| 139 | N-[2-Fluoro-5-[3-[2-(2-pyridyl)vinyl]-1H-indazol-6-ylamino]phenyl]-1,3-dimethyl-1 H-pyrazole-5-carboxamide | |
| 140 | N-(4-tert-Butylphenyl)-N-[2-(cyclopentylamino)-2-oxo-1 -(pyridin-3-yl)ethyl]-1 H-imidazole-4-carboxamide | |
| 141 | 7-[7-[(3bR,4aS)-3-Methyl-8-oxo-4,4a,5,8-tetrahydrocyclopropa[c]pyrrolo[3,2-e]indol-6(1H)-ylcarbonyl]-4-hydroxy-5-methoxy-1,2,3,6-tetrahydropyrrolo[3,2-e]indol-3-ylcarbonyl]-4-hydroxy-5-methoxy-1,2,3,6-tetrahydropyrrolo[3,2-e]indole-3-carboxamide | Rachelmycin |
| 142 | (6bS,8aS,11R,12aR,12bS,14aR)-3-Hydroxy-4,6b,8a, 11, 12b, 14a-hexamethyl-2,6b,7,8,8a,9,10,11,12,12a,12b,13,14,14a-tetradecahydropicene-2,10-dione | Maytenin; Tingenin A; Tingenone |
| 143 | (aR)-(2S)-6-Fluoro-5-[3-[(aS)-8-fluoro-1-methyl-2,4-dioxo-1,4-dihydroquinazolin-3(2H)-yl]-2-methylphenyl]-2-(2-hydroxypropan-2-yl)-2,3,4,9-tetrahydro-1H-carbazole-8-carboxamide | |
| 144 | 2-Benzyl-5-(4-chlorophenyl)-6-[4-(methylsulfanyl)phenyl]pyridazin-3(2H)-one | |
| 145 | 3-[3-Carboxy-2-methyl-1-[(Z)-5,5,8,8-tetramethyl-5,6,7,8-tetrahydroanthracen-2-ylmethylene]-2(Z)-propenyl]benzoic acid; 4-(3-Carboxyphenyl)-3-methyl-5-(5,5,8,8-tetramethyl-5,6,7,8-tetrahydroanthracen-2-yl)-2(Z),4(Z)-pentadienoic acid | |
| 146 | (S)-N-[1-(2-Fluorophenyl)-4-oxo-3,4,6,7-tetrahydropyrrolo[3,2,1 -jk][1 ,4]benzodiazepin-3-yl]-1 H-indole-2-carboxamide | Pranazepide (Prop INN) |
| 147 | 2',6-Difluoro-5'-[3-(1-hydroxy-1-methylethyl)imidazo[1,2-b][1,2,4]triazin-7-yl]biphenyl-2-carbonitrile | |
| 148 | 4-(Dimethylamino)-N-[3-[4-[2-hydroxy-1(S)-phenylethylamino]-6-phenylfuro[2,3-d]pyrimidin-5-yl]phenyl]-2-butenamide | |
| 149 | N'-[4-[4-(Dimethylamino)piperidin-1-ylcarbonyl]phenyl]-N-[4-[4,6-di(4-morpholinyl)-1,3,5-triazin-2-yl]phenyl]urea | Gedatolisib (USAN; Rec INN) |
| 150 | 6-(2-Chlorophenyl)-9-[(4-methoxyphenyl)carbamoyl]-1-methyl-4,7,8,10-tetrahydropyrido[4',3':4,5]thieno[3,2-f][1,2,4]triazolo[4,3-a][1,4]diazepine | Setipafant (Rec INN) |
| 151 | N-(1',2-Dihydroxy-1,2'-binaphthalen-4'-yl)-4-methoxybenzenesulfonamide | |
| 152 | 3'-[2(Z)-[1-(3,4-Dimethylphenyl)-3-methyl-5-oxo-4,5-dihydro-1H-pyrazol-4-ylidene]hydrazino]-2'-hydroxybiphenyl-3-carboxylic acid 2-aminoethanol (1:2) | Eltrombopag olamine (Rec INNM; USAN) |
| 153 | (11beta,16beta)-9-Fluoro-1',4'-dihydro-11,21-dihydroxy-2'H-naphtho[2',3':16,17]pregna-1,4-diene-3,20-dione monohydrate; 9-Fluoro-1',4'-dihydro-11beta,21-dihydroxy-2'betaH-naphtho[2',3':16,17]pregna-1,4-diene-3,20-dione monohydrate | Naflocort (BAN; Rec INN) |
| 154 | N-(3-[5-[(1-Ethylpiperidin-4-yl)(methyl)amino]-3-(pyrimidin-5-yl)-1H-pyrrolo[3,2-b]pyridin-1-yl]-2,4-difluorophenyl)propane-1-sulfonamide | |
| 155 | N-Cycloheptyl-N-(9H-fluoren-2-ylmethyl)-N'-(2,4,6-trimethylphenyl)urea | |
| 156 | 5-[(1 R,3R)-1-(1-Ethyl-1H-pyrazol-4-yl)-3-[4-(5-fluoropyridin-2-yl)-1 H-imidazol-2-yl]-2,3,4,9-tetrahydro-1 H-beta-carbolin-1-yl]-3-methyl-1,3,4-oxadiazol-2(3H)-one | |
| 157 | N-(5-Ethyl-2,4-dimethylphenyl)-4-[3-fluoro-4-(4-methyl-1 H-imidazol-1-yl)phenyl]thiazol-2-amine | |
| 158 | N-[2-(Diethylamino)ethyl]-2,4-dimethyl-5-[2-oxo-5-(2-phenylthiazol-4-yl)-2,3-dihydro-1 H-indol-3-ylidenemethyl]-1 H-pyrrole-3-carboxamide | Amcasertib (USAN; Rec INN) |
| 159 | N-(4-Oxo-3,4-dihydroquinazolin-2-yl)-L-prolyl-N-methyl-L-3-(2-naphthyl)alanine N-benzyl-N-methylamide | |
| 160 | (5S,6R,8R)-3'-(Methylpolyehtyleneglycol)-5-methyl-7,8,14,15-tetrahydro-2'H,4'H,13H-spiro[5,8-epoxy-4b,8a,14-triazadibenzo[b,h]cycloocta[1,2,3,4-jkl]cyclopenta[e]-as-indacene-6,5'-[1,3]oxazolidine]-2',4',13-trione | Pegcantratinib (USAN; Rec INN) |
ii) at least one inert pharmaceutically acceptable excipient.

3. A compound for use according to claim 1 or a pharmaceutical composition for use according to claim 2, wherein said compound or composition is administered directly in the respiratory system.
